# EUROPEAN PATENT SPECIFICATION

(11) **EP 4 120 837 B1**
(45) Date of publication and mention of the grant of the patent: **07.02.2024**
(21) Application number: 20732478.1
(22) Date of filing: 02.06.2020
(51) Int. Cl.: A01N 47/30, C07C 275/42, A01P 21/00

(54) **INHIBITORS OF CYTOKININ OXIDASE DERIVED FROM 2-(3-PHENYLUREIDO)BENZAMIDE, USE THEREOF AND PREPARATIONS CONTAINING THESE DERIVATIVES**
INHIBITOREN DER CYTOKININOXIDASE AUS 2-(3-PHENYLUREIDO)BENZAMID, IHRE VERWENDUNG UND DIESE DERIVATE ENTHALTENDE ZUBEREITUNGEN
INHIBITEURS DE LA CYTOKININE OXYDASE DÉRIVÉS DE 2-(3-PHÉNYLURÉIDO) BENZAMIDE, LEUR UTILISATION ET PRÉPARATIONS CONTENANT CES DÉRIVÉS

(30) Priority: 16.03.2020 CZ 20200143
(43) Date of publication of application: 25.01.2023
(73) Proprietor: Univerzita Palackého v Olomouci, 779 00 Olomouc (CZ)
(72) Inventor: NISLER, Jaroslav, 14000 Praha 4 (CZ); KOPECNY, David, 79604 Prostejov (CZ); SPICHAL, Lukas, 77900 Bystrocice (CZ); STRNAD, Miroslav, 77900 Olomouc (CZ)
(74) Representative: Harber IP s.r.o.
(86) International application number: PCT/CZ2020/050037
(87) International publication number: WO 2021/185390

(56) References cited:
- WO-A1-97/45400
- CN-A- 107 892 598
- CN-B- 106 831 494
- KOPECNY D ET AL: "Phenyl- and benzylurea cytokinins as competitive inhibitors of cytokinin oxidase/dehydrogenase: A structural study", BIOCHIMIE, MASSON, PARIS, FR, vol. 92, no. 8, 1 August 2010 (2010-08-01) , pages 1052-1062, XP027141295, ISSN: 0300-9084 [retrieved on 2010-05-15]
- Issa Yavari ET AL: "Reaction of Primary Alkylamines, Heterocumulenes, and Isatoic Anhydride, Catalyzed by Magnetic Fe 3 O 4 Nanoparticles in H 2 O", Helvetica Chimica Acta, 1 January 2011 (2011-01-01), pages 1825-1830, XP055733685, Retrieved from the Internet: URL:https://onlinelibrary.wiley.com/doi/ab s/10.1002/hlca.201100071 [retrieved on 2020-09-24]
- YUFENG JING ET AL: "Lanthanide-catalyzed cyclocarbonylation and cyclothiocarbonylation: a facile synthesis of benzannulated 1,3-diheteroatom five- and six-membered heterocycles", SCIENCE CHINA CHEMISTRY; THE FRONTIERS OF CHEMICAL BIOLOGY AND SYNTHESIS, vol. 57, no. 8, 21 June 2014 (2014-06-21), pages 1117-1125, XP055733686, Sience China Press ISSN: 1674-7291, DOI: 10.1007/s11426-014-5149-0
- DATABASE REGISTRY [Online] CHEMICAL ABSTRACTS SERVICE, COLUMBUS, OHIO, US; 19 April 2018 (2018-04-19), XP002800466, Database accession no. 2215393-92-1
- DATABASE REGISTRY [Online] CHEMICAL ABSTRACTS SERVICE, COLUMBUS, OHIO, US; 17 April 2018 (2018-04-17), XP002800467, Database accession no. 2214141-23-6
- DATABASE REGISTRY [Online] CHEMICAL ABSTRACTS SERVICE, COLUMBUS, OHIO, US; 15 April 2018 (2018-04-15), XP002800468, Database accession no. 2212617-45-1
- DATABASE REGISTRY [Online] CHEMICAL ABSTRACTS SERVICE, COLUMBUS, OHIO, US; 28 April 2016 (2016-04-28), XP002800469, Database accession no. 1899546-27-0
- DATABASE REGISTRY [Online] CHEMICAL ABSTRACTS SERVICE, COLUMBUS, OHIO, US; 17 September 2010 (2010-09-17), XP002800470, Database accession no. 1241996-30-4
- DATABASE REGISTRY [Online] CHEMICAL ABSTRACTS SERVICE, COLUMBUS, OHIO, US; 10 July 2006 (2006-07-10), XP002800471, Database accession no. 891416-76-5
- DATABASE REGISTRY [Online] CHEMICAL ABSTRACTS SERVICE, COLUMBUS, OHIO, US; 5 July 2006 (2006-07-05), XP002800472, Database accession no. 890572-70-0

## Description

### Technical Field

The present invention relates to substituted 2-(3-phenylureido)benzamide derivatives, their use for increasing the seed yield of crops in agriculture, and preparations containing these derivatives.

### Background Art

Plant hormones cytokinins regulate plant growth and development. Cytokinins have been extensively tested in field conditions and have been shown to increase the yield of agriculturally important crops. Their positive effect was observed in both optimal and stress conditions (summarized in Koprna R. et al., 2016, Bioorg. Med. Chem. 24, 484-492).

Cytokinin oxidase/dehydrogenase (CKX, EC 1.5.99.12) is, an enzyme, that degrades cytokinins in all plants. Thus, inhibition of this enzyme increases the yield of various agriculturally important crops. For example, a fiber content and yield in cotton plants (*Gossypium sp*.)was increased (Zhao J. et al., 2015, Mol. Breed. 35, 60-71). It has also been achieved to improve tillering and to increase total seed yield of rice (Ashikari M. et al., 2005, Science 309, 741-745; Yeh SY et al., 2015, Rice (NY). 8, 36-49). I In the model plant *Arabidopsis thaliana,* inhibition of CKX also leads to an increase in the number of flowers and, subsequently, in the number of siliques, which has led to an increase in overall yield (Bartrina I. et al., 2011, The Plant Cell 23, 69-80). Inhibition of the CKX enzyme leads to an increase in the content of endogenous cytokinins in plants, which has a positive effect on their yield.

The inhibitory strength of a CKX inhibitor is very important. The stronger the inhibitor is, the smaller amount of it is needed in the field to achieve the desired effect. This would also have a positive environmental impact. For this reason, there is a need to provide new groups of compounds that have a higher inhibitory potency.

Several groups of compounds having CKX inhibitory activity have been developed in the past. These include in particular anilinopurines (Zatloukal M. et al., 2008, Bioorg. Med. Chem. 16, 9268-9275), thidiazuron and its derivatives (Nisler J. et al., 2016, Plant Mol. Biol. 92, 235 -248) or 1-(2-chlor-pyridin-4-yl)-3-phenylurea = CPPU and its derivatives (Kopečný D. et al., 2010, Biochimie 92, 1052-62). Other structurally similar substances are also disclosed in patent documents CN106831494B and CN107892598A.

### Disclosure of the Invention

The present invention relates to derivatives of 2-(3-phenylureido)benzamide, showing the highest known inhibitory activity against cytokinin oxidase/dehydrogenase (CKX, EC 1.5.99.12). Their application to plants leads to higher yields in both stress and optimal conditions.

The object of the present invention a compound of the general formula (I), 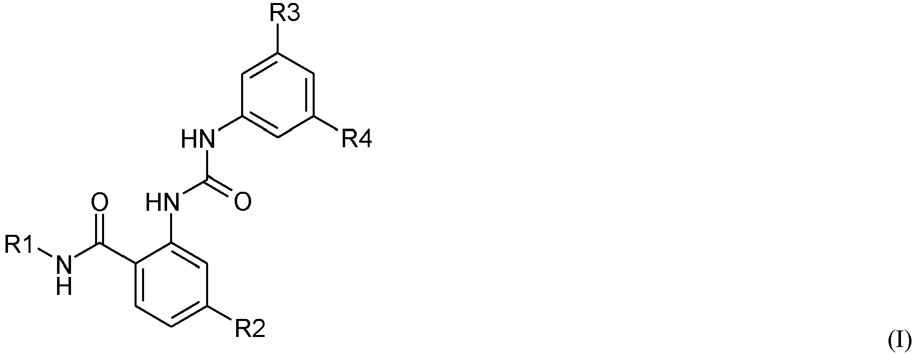 wherein
R1 is selected from the group consisting of hydrogen, amino group, C₁-C₅ alkyl, C₂-C₅ alkenyl, C₂-C₅ alkynyl, wherein C₁-C₅ alkyl can be optionally substituted with hydroxy and/or amino group;
R2 is selected from the group consisting of hydrogen, halogen, methoxy group or halogenated methoxy group; wherein the halogenated methoxy group is preferably trifluoromethoxy group; R3 is selected from the group consisting of hydrogen, halogen, methyl, methoxy group or trifluoromethoxy group;
R4 is selected from the group consisting of halogen, methyl, methoxy group and trifluoromethoxy group;
with the proviso that 2-[3-(3-chlorophenyl)ureido]benzamide is excluded.

The generic substituent groups have meanings as defined herein below:
alkyl denotes branched or linear alkyl group;
(C₁-C₅)alkyl denotes branched or linear alkyl group with 1 up to 5 carbon atoms;
alkenyl denotes branched or linear hydrocarbon chain with at least one double bond;
alkynyl denotes branched or linear hydrocarbon chain with at least one triple bond;
halogen is selected from the group comprising fluorine, chlorine, bromine and
iodine atom;
hydroxy denotes the group -OH;
amino denotes the group -NH₂;
methoxy denotes the group -O-CH₃;
halogenated methoxy group is -O-halogenated methyl, and optionally it is trifluoromethoxy group -O-CF₃.

If the compounds falling into the scope of this invention contain a chiral centre, then all their enantiomers, mixtures of enantiomers and racemates fall within the framework of the present invention. The present invention is further meant to include the compounds of general formula (I) in the form of salts with alkali metals, ammonium or amines, as well as in the form of addition salts with acids.

In one preferred embodiment R1 is selected from the group consisting of hydrogen, methyl, ethyl, propyl, -NH₂, 2-hydoxyethyl, 3-hydoxypropyl and 2-aminoethyl.

In one preferred embodiment R2 is selected from the group consisting of hydrogen, fluorine, chlorine, bromine, methoxy group and trifluoromethoxy group.

In one preferred embodiment at least one of the R3 and R4 is halogen.

Preferably both R3 and R4 are halogenes, selected independently from the group comprising F, Cl, Br,
or one of R3 and R4 is halogen and the other is selected from the group consisting of hydrogen, methyl, methoxy group or trifluoromethoxy group.

In one preferred embodiment R1 is selected from the group consisting of H, -CH₃, -CH₂CH₃, - CH₂CH₂CH₃, -NH₂, -CH₂CH₂(OH), -CH₂CH₂CH₂(OH) and -CH₂CH₂(NH₂);
R2 is selected from the group consisting of H, F, Cl, Br, -OCH₃ and -OCF₃;
and R3 and R4 are both halogenes, selected independently from the group consisting of F, Cl and
Br, or one of R3 and R4 is halogen and the other is selected from the group consisting of H, -CH₃, -OCH₃ or -OCF₃.

In one preferred embodiment, the compound of the general formula (I) are selected from the group comprising:
**1. R1** is **hydrogen** and
   **R2-H:** 2-[3-(3-fluorophenyl)ureido]benzamide, 2-[3-(3-bromophenyl)ureido]benzamide, 2-[3-(3,5-difluorophenyl)ureido]benzamide, 2-[3-(3,5-dichlorophenyl)ureido]benzamide, 2-[3-(3,5-dibromophenyl)ureido]benzamide, 2-[3-(3-chloro-5-fluorophenyl)ureido]benzamide, 2-[3-(3-bromo-5-chlorophenyl)ureido]benzamide, 2-[3-(3-chloro-5-methylphenyl)ureido]benzamide, 2-[3-(3-chloro-5-trifluoromethoxyphenyl)ureido]benzamide, 2-[3-(3-chloro-5-methoxyphenyl)ureido]benzamide,
   **R2-F:** 2-[3-(3-fluorophenyl)ureido]-4-fluorobenzamide, 2-[3-(3-chlorophenyl)ureido]-4-fluorobenzamide, 2-[3-(3-bromophenyl)ureido]-4-fluorobenzamide, 2-[3-(3,5-difluorophenyl)ureido]-4-fluorobenzamide, 2-[3-(3,5-dichlorophenyl)ureido]-4-fluorobenzamide, 2-[3-(3,5-dibromophenyl)ureido]-4-fluorobenzamide, 2-[3-(3-chloro-5-fluorophenyl)ureido]-4-fluorobenzamide, 2-[3-(3-bromo-5-chlorophenyl)ureido]-4-fluorobenzamide, 2-[3-(3-chloro-5-methylphenyl)ureido]-4-fluorobenzamide, 2-[3-(3-chloro-5-trifluoromethoxyphenyl)ureido]-4-fluorobenzamide, 2-[3-(3-chloro-5-methoxyphenyl)ureido]-4-fluorobenzamide,
   **R2-Cl:** 4-chloro-2-[3-(3-fluorophenyl)ureido]benzamide, 4-chloro-2-[3-(3-chlorophenyl)ureido]benzamide, 4-chloro-2-[3-(3-bromophenyl)ureido]benzamide, 4-chloro-2-[3-(3,5-difluorophenyl)ureido]benzamide, 4-chloro-2-[3-(3,5-dichlorophenyl)ureido]benzamide, 4-chloro-2-[3-(3,5-dibromophenyl)ureido]benzamide, 2-[3-(3-chloro-5-fluorophenyl)ureido]-4-chlorobenzamide, 2-[3-(3-bromo-5-chlorophenyl)ureido]-4-chlorobenzamide, 2-[3-(3-chloro-5-trifluoromethoxyphenyl)ureido]-4-chlorobenzamide, 2-[3-(3-chloro-5-methoxyphenyl)ureido]-4-chlorobenzamide, 2-[3-(3-chloro-5-methylphenyl)ureido]-4-chlorobenzamide,
   **R2-Br:** 4-bromo-2-[3-(3-fluorophenyl)ureido]benzamide, 4-bromo-2-[3-(3-chlorophenyl)ureido]benzamide, 4-bromo-2-[3-(3-bromophenyl)ureido]benzamide, 4-brom-2-[3-(3,5-difluorophenyl)ureido]benzamide, 4-bromo-2-[3-(3,5-dichlorophenyl)ureido]benzamide, 4-bromo-2-[3-(3,5-dibromophenyl)ureido]benzamide, 4-bromo-2-[3-(3-chloro-5-fluorophenyl)ureido] benzamide, 2-[3-(3-bromo-5-chlorophenyl)ureido]-4-bromobenzamide, 4-bromo-2-[3-(3-chloro-5-trifluoromethoxyphenyl)ureido]benzamide, 4-bromo-2-[3-(3-chloro-5-methoxyphenyl)ureido]benzamide, 4-bromo-2-[3-(3-chloro-5-methylphenyl)ureido]benzamide,
   **R2-OCH₃:** 2-[3-(3-fluorophenyl)ureido]-4-methoxybenzamide, 2-[3-(3-chlorophenyl)ureido]-4-methoxybenzamide, 2-[3-(3-bromophenyl)ureido]-4-methoxybenzamide, 2-[3-(3,5-difluorophenyl)ureido]-4-methoxybenzamide, 2-[3 -(3,5-dichlorophenyl)ureido]-4-methoxybenzamide, 2-[3-(3,5-dibromophenyl)ureido]-4-methoxybenzamide, 2-[3-(3-chloro-5-fluorophenyl)ureido]-4-methoxybenzamide, 2-[3-(3-bromo-5-chlorophenyl)ureido]-4-methoxybenzamide, 2-[3-(3-chloro-5-methylphenyl)ureido]-4-methoxybenzamide,
   **R2-OCF₃**: 2-[3-(3-fluorophenyl)ureido]-4-trifluoromethoxybenzamide, 2-[3-(3-chlorophenyl)ureido]-4-trifluoromethoxybenzamide, 2-[3-(3-bromophenyl)ureido]-4-trifluoromethoxybenzamide, 2-[3-(3,5-difluorophenyl)ureido]-4-trifluoromethoxybenzamide, 2-[3-(3,5-dichlorophenyl)ureido]-4-trifluoromethoxybenzamide, 2-[3-(3,5-dibromophenyl)ureido]-4-trifluoromethoxybenzamide, 2-[3-(3-chloro-5-fluorophenyl)ureido]-4-trifluoromethoxybenzamide, 2-[3-(3-bromo-5-chlorophenyl)ureido]-4-trifluoromethoxybenzamide; 2-[3-(3-chloro-5-methylphenyl)ureido]-4-trifluoromethoxybenzamide,
**2. R1** is **NH₂** and
   **R2-H:** 1-(3-fluorophenyl)-3-(2-hydrazinocarbonylphenyl)-urea, 1-(3-chlorophenyl)-3-(2-hydrazinocarbonylphenyl)-urea, 1-(3-bromophenyl)-3-(2-hydrazinocarbonylphenyl)-urea, 1-(3,5-difluorophenyl)-3-(2-hydrazinocarbonylphenyl)-urea, 1-(3,5-dichlorophenyl)-3-(2-hydrazinocarbonylphenyl)-urea, 1-(3,5-dibromophenyl)-3-(2-hydrazinocarbonylphenyl)-urea, 1-(3-chloro-5-fluorophenyl)-3-(2-hydrazinocarbonylphenyl)-urea, 1-(3-bromo-5-chlorophenyl)-3-(2-hydrazinocarbonylphenyl)-urea,
   **R2-F:** 1-(3-fluorophenyl)-3-(5-fluoro-2-hydrazinocarbonylphenyl)-urea, 1-(3-chlorophenyl)-3-(5-fluoro-2-hydrazinocarbonylphenyl)-urea, 1-(3-bromophenyl)-3-(5-fluoro-2-hydrazinocarbonylphenyl)-urea, 1-(3,5-difluorophenyl)-3-(5-fluoro-2-hydrazinocarbonylphenyl)-urea, 1-(3,5-dichlorophenyl)-3-(5-fluoro-2-hydrazinocarbonylphenyl)-urea, 1-(3,5-dibromophenyl)-3-(5-fluoro-2-hydrazinocarbonylphenyl)-urea, 1-(3-chloro-5-fluorophenyl)-3-(5-fluoro-2-hydrazinocarbonylphenyl)-urea, 1-(3-bromo-5-chlorophenyl)-3-(5-fluoro-2-hydrazinocarbonylphenyl)-urea,
   **R2-Cl:** 1-(5-chloro-2-hydrazinocarbonylphenyl)-3-(3-fluorophenyl)-urea, 1-(5-chloro-2-hydrazinocarbonylphenyl)-3-(3-chlorophenyl)-urea, 1-(3-bromophenyl)-3-(5-chloro-2-hydrazinocarbonylphenyl)-urea, 1-(5-chloro-2-hydrazinocarbonylphenyl)-3-(3,5-difluorophenyl)-urea, 1-(5-chloro-2-hydrazinocarbonylphenyl)-3-(3,5-dichlorophenyl)-urea, 1-(3,5-dibromophenyl)-3-(5-chloro-2-hydrazinocarbonylphenyl)-urea, 1-(3-chloro-5-fluorophenyl)-3-(5-chloro-2-hydrazinocarbonylphenyl)-urea, 1-(3-bromo-5-chlorophenyl)-3-(5-chloro-2-hydrazinocarbonylphenyl)-urea
   **R2-Br:** 1-(5-bromo-2-hydrazinocarbonylphenyl)-3-(3-fluorophenyl)-urea, 1-(5-bromo-2-hydrazinocarbonylphenyl)-3-(3-chlorophenyl)-urea, 1-(5-bromo-2-hydrazinocarbonylphenyl)-3-(3-bromophenyl)-urea, 1-(5-bromo-2-hydrazinocarbonylphenyl)-3-(3,5-difluorophenyl)-urea, 1-(5-bromo-2-hydrazinocarbonylphenyl)-3-(3,5-dichlorophenyl)-urea, 1-(5-bromo-2-hydrazinocarbonylphenyl)-3-(3,5-dibromophenyl)-urea, 1-(5-bromo-2-hydrazinocarbonylphenyl)-3-(3-chloro-5-fluorophenyl)-urea, 1-(5-bromo-2-hydrazinocarbonylphenyl)-3-(3-bromo-5-chlorophenyl)-urea,
   **R2-OCH₃:** 1-(3-fluorophenyl)-3-(2-hydrazinocarbonyl-5-methoxyphenyl)-urea, 1-(3-chlorophenyl)-3-(2-hydrazinocarbonyl-5-methoxyphenyl)-urea, 1-(3-bromophenyl)-3-(2-hydrazinocarbonyl-5-methoxyphenyl)-urea, 1-(3,5-difluorophenyl)-3-(2-hydrazinocarbonyl-5-methoxyphenyl)-urea, 1-(3,5-dichlorophenyl)-3-(2-hydrazinocarbonyl-5-methoxyphenyl)-urea, 1-(3,5-dibromophenyl)-3-(2-hydrazinocarbonyl-5-methoxyphenyl)-urea, 1-(3-chloro-5-fluorophenyl)-3-(2-hydrazinocarbonyl-5-methoxyphenyl)-urea, 1-(3-bromo-5-chlorophenyl)-3-(2-hydrazinocarbonyl-5-methoxyphenyl)-urea,
   **R2-OCF₃**: 1-(3-fluorophenyl)-3-(2-hydrazinocarbonyl-5-trifluoromethoxyphenyl)-urea, 1-(3-chlorophenyl)-3-(2-hydrazinocarbonyl-5-trifluoromethoxyphenyl)-urea, 1-(3-bromophenyl) -3-(2-hydrazinocarbonyl-5-trifluoromethoxyphenyl)-urea, 1-(3,5-difluorophenyl)-3-(2-hydrazinocarbonyl-5-trifluoromethoxyphenyl)-urea, 1-(3,5-dichlorophenyl)-3-(2-hydrazinocarbonyl-5-trifluoromethoxyphenyl)-urea, 1-(3,5-dibromophenyl)-3-(2-hydrazinocarbonyl-5-trifluoromethoxyphenyl)-urea, 1-(3-chloro-5-fluorophenyl)-3-(2-hydrazinocarbonyl-5-tri-fluoromethoxyphenyl)-urea, 1-(3-bromo-5-chlorophenyl)-3-(2-hydrazinocarbonyl-5-trifluoromethoxyphenyl)-urea;
**3. R1** is **methyl** and
   **R2-H:** 2-[3-(3-fluorophenyl)ureido]-*N*-methylbenzamide, 2-[3-(3-chlorophenyl)ureido]-*N-*methylbenzamide, 2-[3-(3-bromophenyl)ureido]-*N*-methylbenzamide, 2-[3-(3,5-difluorophenyl)ureido]-*N*-methylbenzamide, 2-[3-(3,5-dichlorophenyl)ureido]-*N-*methylbenzamide, 2-[3-(3,5-dibromophenyl)ureido]-*N*-methylbenzamide, 2-[3-(3-chloro-5-fluorophenyl)ureido]-*N*-methylbenzamide, 2-[3-(3-bromo-5-chlorophenyl)ureido]-*N-*methylbenzamide,
   **R2-F:** 2-[3-(3-fluorophenyl)ureido]-4-fluoro-*N*-methylbenzamide, 2-[3-(3-chlorophenyl)ureido]-4-fluoro-*N*-methylbenzamide, 2-[3-(3-bromophenyl)ureido]-4-fluoro-*N*-methylbenzamide, 2-[3-(3,5-difluorophenyl)ureido]-4-fluoro-*N*-methylbenzamide, 2-[3-(3,5-dichlorophenyl)ureido]-4-fluoro-*N*-methylbenzamide, 2-[3-(3,5-dibromophenyl)ureido]-4-fluoro-*N*-methylbenzamide, 2-[3-(3-chloro-5-fluorophenyl)ureido]-4-fluoro-*N*-methylbenzamide, 2-[3-(3-bromo-5-chlorophenyl)ureido]-4-fluoro-*N*-methylbenzamide,
   **R2-Cl:** 4-chloro-2-[3-(3-fluorophenyl)ureido]-*N*-methylbenzamide, 4-chloro-2-[3-(3-chlorophenyl)ureido]-*N*-methylbenzamide, 4-chloro-2-[3-(3-bromophenyl)ureido]-*N-*methylbenzamide, 4-chloro-2-[3-(3,5-difluorophenyl)ureido]-*N*-methylbenzamide, 4-chloro-2-[3-(3,5-dichlorophenyl)ureido]-*N*-methylbenzamide, 4-chloro-2-[3-(3,5-dibromophenyl)ureido]-*N-*methylbenzamide, 2-[3-(3-chloro-5-fluorophenyl)ureido]-4-chloro-*N*-methylbenzamide, 2-[3-(3-bromo-5-chlorophenyl)ureido]-4-chloro-*N*-methylbenzamide,
   **R2-Br:** 4-bromo-2-[3-(3-fluorophenyl)ureido]-*N*-methylbenzamide, 4-bromo-2-[3-(3-chlorophenyl)ureido]-*N*-methylbenzamide, 4-bromo-2-[3-(3-bromophenyl)ureido]-*N-*methylbenzamide, 4-brom-2-[3-(3,5-difluorophenyl)ureido]-N methylbenzamide, 4-bromo-2-[3-(3,5-dichlorophenyl)ureido]-*N*-methylbenzamide, 4-bromo-2-[3-(3,5-dibromophenyl)ureido]-*N-*methylbenzamide, 4-bromo-2-[3-(3-chloro-5-fluorophenyl)ureido]-*N*-methylbenzamide, 2-[3-(3-bromo-5-chlorophenyl)ureido]-4-bromo-*N*-methylbenzamide,
   **R2-OCH₃:** 2-[3-(3-fluorophenyl)ureido]-4-methoxy-*N-*methylbenzamide, 2-[3-(3-chlorophenyl)ureido]-4-methoxy-*N*-methylbenzamide, 2-[3-(3-bromophenyl)ureido]-4-methoxy-*N*-methylbenzamide, 2-[3-(3,5-difluorophenyl)ureido]-4-methoxy-*N*-methylbenzamide, 2-[3-(3,5-dichlorophenyl)ureido]-4-methoxy-*N*-methylbenzamide, 2-[3-(3,5-dibromophenyl)ureido]-4-methoxy-*N*-methylbenzamide, 2-[3-(3-chloro-5-fluorophenyl)ureido]-4-methoxy-*N-*methylbenzamide, 2-[3-(3-bromo-5-chlorophenyl)ureido]-4-methoxy-*N*-methylbenzamide, **R2-OCF₃**: 2-[3-(3-fluorophenyl)ureido]-4-trifluoromethoxy-*N-*methylbenzamide, 2-[3-(3-chlorophenyl)ureido]-4-trifluoromethoxy-*N*-methylbenzamide, 2-[3-(3-bromophenyl)ureido]-4-trifluoromethoxy-*N*-methylbenzamide, 2-[3-(3,5-difluorophenyl)ureido]-4-trifluoromethoxy-*N-*methylbenzamide, 2-[3-(3,5-dichlorophenyl)ureido]-4-trifluoromethoxy-*N*-methylbenzamide, 2-[3-(3,5-dibromophenyl)ureido]-4-trifluoromethoxy-*N*-methylbenzamide, 2-[3-(3-chloro-5-fluorophenyl)ureido]-4-trifluoromethoxy-*N*-methylbenzamide, 2-[3-(3-bromo-5-chlorophenyl)ureido]-4-trifluoromethoxy-*N*-methylbenzamide;
**4. R1** is **ethyl** and
   **R2-H:** 2-[3-(3-fluorophenyl)ureido]-*N*-ethylbenzamide, 2-[3-(3-chlorophenyl)ureido]-*N-*ethylbenzamide, 2-[3-(3-bromophenyl)ureido]-*N*-ethylbenzamide, 2-[3-(3,5-difluorophenyl)ureido]-*N*-ethylbenzamide, 2-[3-(3,5-dichlorophenyl)ureido]-*N*-ethylbenzamide, 2-[3-(3,5-dibromophenyl)ureido]-*N*-ethylbenzamide, 2-[3-(3-chloro-5-fluorophenyl)ureido]-*N-*ethylbenzamide, 2-[3-(3-bromo-5-chlorophenyl)ureido]-*N*-ethylbenzamide,
   **R2-F:** 2-[3-(3-fluorophenyl)ureido]-4-fluoro-*N*-ethylbenzamide, 2-[3-(3-chlorophenyl)ureido]-4-fluoro-*N*-ethylbenzamide, 2-[3-(3-bromophenyl)ureido]-4-fluoro-*N*-ethylbenzamide, 2-[3-(3,5-difluorophenyl)ureido]-4-fluoro-*N*-ethylbenzamide, 2-[3-(3,5-dichlorophenyl)ureido]-4-fluoro-*N*-ethylbenzamide, 2-[3-(3,5-dibromophenyl)ureido]-4-fluoro-*N*-ethylbenzamide, 2-[3-(3-chloro-5-fluorophenyl)ureido]-4-fluoro-*N*-ethylbenzamide, 2-[3-(3-bromo-5-chlorophenyl)ureido]-4-fluoro-*N*-ethylbenzamide,
   **R2-Cl:** 4-chloro-2-[3-(3-fluorophenyl)ureido]-N ethylbenzamide, 4-chloro-2-[3-(3-chlorophenyl)ureido]-*N*-ethylbenzamide, 4-chloro-2-[3-(3-bromophenyl)ureido]-*N-*ethylbenzamide, 4-chloro-2-[3-(3,5-difluorophenyl)ureido]-*N*-ethylbenzamide, 4-chloro-2-[3-(3,5-dichlorophenyl)ureido]-*N*-ethylbenzamide, 4-chloro-2-[3-(3,5-dibromophenyl)ureido]-*N-*ethylbenzamide, 2-[3-(3-chloro-5-fluorophenyl)ureido]-4-chloro-*N*-ethylbenzamide, 2-[3-(3-bromo-5-chlorophenyl)ureido]-4-chloro*-N-*ethylbenzamide,
   **R2-Br:** 4-bromo-2-[3-(3-fluorophenyl)ureido]-*N*-ethylbenzamide, 4-bromo-2-[3-(3-chlorophenyl)ureido]-*N*-ethylbenzamide, 4-bromo-2-[3-(3-bromophenyl)ureido]-*N-*ethylbenzamide, 4-brom-2-[3-(3,5-difluorophenyl)ureido]-*N*-ethylbenzamide, 4-bromo-2-[3-(3,5-dichlorophenyl)ureido]-*N*-ethylbenzamide, 4-bromo-2-[3-(3,5-dibromophenyl)ureido]-*N-*ethylbenzamide, 4-bromo-2-[3-(3-chloro-5-fluorophenyl)ureido] -*N*-ethylbenzamide, 2-[3-(3-bromo-5-chlorophenyl)ureido]-4-bromo-*N*-ethylbenzamide,
   **R2-OCH₃:** 2-[3-(3-fluorophenyl)ureido]-4-methoxy-*N*-ethylbenzamide, 2-[3-(3-chlorophenyl)ureido]-4-methoxy-*N*-ethylbenzamide, 2-[3-(3-bromophenyl)ureido]-4-methoxy-*N*-ethylbenzamide, 2-[3-(3,5-difluorophenyl)ureido]-4-methoxy-*N*-ethylbenzamide, 2-[3-(3,5-dichlorophenyl)ureido]-4-methoxy-*N*-ethylbenzamide, 2-[3-(3,5-dibromophenyl)ureido]-4-methoxy-*N*-ethylbenzamide, 2-[3-(3-chloro-5-fluorophenyl)ureido]-4-methoxy-*N-*ethylbenzamide, 2-[3-(3-bromo-5-chlorophenyl)ureido]-4-methoxy-*N*-ethylbenzamide,
   **R2-OCF₃**: 2-[3-(3-fluorophenyl)ureido]-4-trifluoromethoxy-*N*-ethylbenzamide, 2-[3-(3-chlorophenyl)ureido]-4-trifluoromethoxy-*N*-ethylbenzamide, 2-[3-(3-bromophenyl)ureido]-4-trifluoromethoxy-*N*-ethylbenzamide, 2-[3-(3,5-difluorophenyl)ureido]-4-trifluoromethoxy-*N-*ethylbenzamide, 2-[3-(3,5-dichlorophenyl)ureido]-4-trifluoromethoxy-*N*-ethylbenzamide, 2-[3-(3,5-dibromophenyl)ureido]-4-trifluoromethoxy-*N*-ethylbenzamide, 2-[3-(3-chloro-5-fluorophenyl)ureido]-4-trifluoromethoxy-*N*-ethylbenzamide, 2-[3-(3-bromo-5-chlorophenyl)ureido]-4-trifluoromethoxy-*N*-ethylbenzamide;
**5. R1** is **propyl** and
   **R2-H**: 2-[3-(3-fluorophenyl)ureido]-*N-*propylbenzamide, 2-[3-(3-chlorophenyl)ureido]-*N-*propylbenzamide, 2-[3-(3-bromophenyl)ureido]-*N*-propylbenzamide, 2-[3-(3,5-difluorophenyl)ureido]-*N*-propylbenzamide, 2-[3-(3,5-dichlorophenyl)ureido]-*N-*propylbenzamide, 2-[3-(3,5-dibromophenyl)ureido]-*N*-propylbenzamide, 2-[3-(3-chloro-5-fluorophenyl)ureido]-*N*-propylbenzamide, 2-[3-(3-bromo-5-chlorophenyl)ureido]-*N-*propylbenzamide,
   **R2-F:** 2-[3-(3-fluorophenyl)ureido]-4-fluoro-*N*-propylbenzamide, 2-[3-(3-chlorophenyl)ureido]-4-fluoro-*N*-propylbenzamide, 2-[3-(3-bromophenyl)ureido]-4-fluoro-*N*-propylbenzamide, 2-[3-(3,5-difluorophenyl)ureido]-4-fluoro-*N*-propylbenzamide, 2-[3-(3,5-dichlorophenyl)ureido]-4-fluoro-*N*-propylbenzamide, 2-[3-(3,5-dibromophenyl)ureido]-4-fluoro-*N*-propylbenzamide, 2-[3-(3-chloro-5-fluorophenyl)ureido]-4-fluoro-N propylbenzamide, 2-[3-(3-bromo-5-chlorophenyl)ureido]-4-fluoro-*N*-propylbenzamide,
   **R2-Cl:** 4-chloro-2-[3 -(3 -fluorophenyl)ureido]-*N*-propylbenzamide, 4-chloro-2-[3 -(3 - chlorophenyl)ureido]-*N*-propylbenzamide, 4-chloro-2-[3-(3-bromophenyl)ureido]-*N-*propylbenzamide, 4-chloro-2-[3-(3,5-difluorophenyl)ureido]-*N*-propylbenzamide, 4-chloro-2-[3-(3,5-dichlorophenyl)ureido]-*N*-propylbenzamide, 4-chloro-2-[3-(3,5-dibromophenyl)ureido]-*N-*propylbenzamide, 2-[3-(3-chloro-5-fluorophenyl)ureido]-4-chloro-*N*-propylbenzamide, 2-[3-(3-bromo-5-chlorophenyl)ureido]-4-chloro N propylbenzamide,
   **R2-Br:** 4-bromo-2-[3-(3-fluorophenyl)ureido]-*N*-propylbenzamide, 4-bromo-2-[3-(3-chlorophenyl)ureido]-*N*-propylbenzamide, 4-bromo-2-[3-(3-bromophenyl)ureido]-*N-*propylbenzamide, 4-brom-2-[3-(3,5-difluorophenyl)ureido]-*N*-propylbenzamide, 4-bromo-2-[3-(3,5-dichlorophenyl)ureido]-*N*-propylbenzamide, 4-bromo-2-[3-(3,5-dibromophenyl)ureido]-*N-*propylbenzamide, 4-bromo-2-[3-(3-chloro-5-fluorophenyl)ureido] -*N*-propylbenzamide, 2-[3-(3-bromo-5-chlorophenyl)ureido]-4-bromo-*N*-propylbenzamide,
   **R2-OCH₃:** 2-[3 -(3 -fluorophenyl)ureido]-4-methoxy-*N*-propylbenzamide, 2-[3 -(3 - chlorophenyl)ureido]-4-methoxy-*N*-propylbenzamide, 2-[3-(3-bromophenyl)ureido]-4-methoxy-N propylbenzamide, 2- [3 -(3,5 -difluorophenyl)ureido] -4-methoxy-*N*-propylbenzamide, 2- [3 -(3,5-dichlorophenyl)ureido]-4-methoxy-*N*-propylbenzamide, 2-[3-(3,5-dibromophenyl)ureido]-4-methoxy-*N*-propylbenzamide, 2-[3-(3-chloro-5-fluorophenyl)ureido]-4-methoxy-*N* propylbenzamide, 2-[3-(3-bromo-5-chlorophenyl)ureido]-4-methoxy-*N*-propylbenzamide, **R2-OCF₃**: 2-[3-(3-fluorophenyl)ureido]-4-trifluoromethoxy-*N*-propylbenzamide, 2-[3 -(3 - chlorophenyl)ureido]-4-trifluoromethoxy-*N*-propylbenzamide, 2-[3-(3-bromophenyl)ureido]-4-trifluoromethoxy-*N*-propylbenzamide, 2-[3-(3,5-difluorophenyl)ureido]-4-trifluoromethoxy-*N-*propylbenzamide, 2-[3-(3,5-dichlorophenyl)ureido]-4-trifluoromethoxy-*N*-propylbenzamide, 2-[3-(3,5-dibromophenyl)ureido]-4-trifluoromethoxy-*N*-propylbenzamide, 2-[3-(3-chloro-5-fluorophenyl)ureido]-4-trifluoromethoxy-*N*-propylbenzamide, 2-[3-(3-bromo-5-chlorophenyl)ureido]-4-trifluoromethoxy-*N*-propylbenzamide;
**6. R1** is **-CH₂CH₂OH** and
   **R2-H**: 2-[3-(3-fluorophenyl)ureido]-*N*-(2-hydroxy-ethyl)benzamide, 2-[3-(3-chlorophenyl)ureido]-*N*-(2-hydroxy-ethyl)benzamide, 2-[3-(3-bromophenyl)ureido]-*N*-(2-hydroxy-ethyl)benzamide, 2-[3-(3,5-difluorophenyl)ureido]-*N*-(2-hydroxy-ethyl)benzamide, 2-[3-(3,5-dichlorophenyl)ureido]-*N*-(2-hydroxy-ethyl)benzamide, 2-[3-(3,5-dibromophenyl)ureido]-*N*-(2-hydroxy-ethyl)benzamide, 2-[3-(3-chloro-5-fluorophenyl)ureido]-*N*-(2-hydroxy-ethyl)benzamide, 2-[3-(3-bromo-5-chlorophenyl)ureido]-*N*-(2-hydroxyethyl)benzamide,
   **R2-F:** 2-[3 -(3 -fluorophenyl)ureido]-4-fluoro-N (2-hydroxy-ethyl)benzamide, 2-[3 -(3 - chlorophenyl)ureido]-4-fluoro-*N*-(2-hydroxy-ethyl)benzamide, 2-[3-(3-bromophenyl)ureido]-4-fluoro-*N*-(2-hydroxy-ethyl)benzamide, 2-[3-(3,5-difluorophenyl)ureido]-4-fluoro-*N*-(2-hydroxyethyl)benzamide, 2-[3-(3,5-dichlorophenyl)ureido]-4-fluoro-*N*-(2-hydroxy-ethyl)benzamide, 2-[3-(3,5-dibromophenyl)ureido]-4-fluoro-*N*-(2-hydroxy-ethyl)benzamide, 2-[3-(3-chloro-5-fluorophenyl)ureido]-4-fluoro-*N*-(2-hydroxy-ethyl)benzamide, 2-[3-(3-bromo-5-chlorophenyl)ureido]-4-fluoro-*N*-(2-hydroxy-ethyl)benzamide,
   **R2-Cl:** 4-chloro-2-[3-(3-fluorophenyl)ureido]-*N*-(2-hydroxy-ethyl)benzamide, 4-chloro-2-[3-(3-chlorophenyl)ureido]-*N*-(2-hydroxy-ethyl)benzamide, 4-chloro-2-[3-(3-bromophenyl)ureido]-*N-*(2-hydroxy-ethyl)benzamide, 4-chloro-2-[3-(3,5-difluorophenyl)ureido]-*N*-(2-hydroxyethyl)benzamide, 4-chloro-2-[3-(3,5-dichlorophenyl)ureido]-*N*-(2-hydroxy-ethyl)benzamide, 4-chloro-2-[3-(3,5-dibromophenyl)ureido]-*N*-(2-hydroxy-ethyl)benzamide, 2-[3-(3-chloro-5-fluorophenyl)ureido]-4-chloro-*N*-(2-hydroxy-ethyl)benzamide, 2-[3-(3-bromo-5-chlorophenyl)ureido]-4-chloro-*N*-(2-hydroxy-ethyl)benzamide,
   **R2-Br:** 4-bromo-2-[3-(3-fluorophenyl)ureido]-*N*-(2-hydroxy-ethyl)benzamide, 4-bromo-2-[3-(3-chlorophenyl)ureido]-*N*-(2-hydroxy-ethyl)benzamide, 4-bromo-2-[3-(3-bromophenyl)ureido]-*N-*(2-hydroxy-ethyl)benzamide, 4-brom-2-[3-(3,5-difluorophenyl)ureido]-*N*-(2-hydroxyethyl)benzamide, 4-bromo-2-[3-(3,5-dichlorophenyl)ureido]-*N*-(2-hydroxy-ethyl)benzamide, 4-bromo-2-[3-(3,5-dibromophenyl)ureido]-*N*-(2-hydroxy-ethyl)benzamide, 4-bromo-2-[3-(3-chloro-5-fluorophenyl)ureido] -*N*-(2-hydroxy-ethyl)benzamide, 2-[3-(3-bromo-5-chlorophenyl)ureido]-4-bromo-*N*-(2-hydroxy-ethyl)benzamide,
   **R2-OCH₃:** 2-[3-(3-fluorophenyl)ureido]-4-methoxy-*N*-(2-hydroxy-ethyl)benzamide, 2-[3-(3-chlorophenyl)ureido]-4-methoxy-*N-*(2-hydroxy-ethyl)benzamide, 2-[3-(3-bromophenyl)ureido]-4-methoxy-*N*-(2-hydroxy-ethyl)benzamide, 2-[3-(3,5-difluorophenyl)ureido]-4-methoxy-*N*-(2-hydroxy-ethyl)benzamide, 2-[3-(3,5-dichlorophenyl)ureido]-4-methoxy-*N*-(2-hydroxyethyl)benzamide, 2-[3-(3,5-dibromophenyl)ureido]-4-methoxy-*N*-(2-hydroxy-ethyl)benzamide, 2-[3-(3-chloro-5-fluorophenyl)ureido]-4-methoxy-*N*-(2-hydroxy-ethyl)benzamide, 2-[3-(3-bromo-5-chlorophenyl)ureido]-4-methoxy-*N*-(2-hydroxy-ethyl)benzamide,
   **R2-OCF₃**: 2-[3-(3-fluorophenyl)ureido]-4-trifluoromethoxy-*N*-(2-hydroxy-ethyl)benzamide, 2-[3-(3-chlorophenyl)ureido]-4-trifluoromethoxy-*N*-(2-hydroxy-ethyl)benzamide, 2-[3-(3-bromophenyl)ureido]-4-trifluoromethoxy-*N*-(2-hydroxy-ethyl)benzamide, 2-[3-(3,5-difluorophenyl)ureido]-4-trifluoromethoxy-*N*-(2-hydroxy-ethyl)benzamide, 2-[3-(3,5-dichlorophenyl)ureido]-4-trifluoromethoxy-*N*-(2-hydroxy-ethyl)benzamide, 2-[3-(3,5-dibromophenyl)ureido]-4-trifluoromethoxy-*N*-(2-hydroxy-ethyl)benzamide, 2-[3-(3-chloro-5-fluorophenyl)ureido]-4-trifluoromethoxy-*N*-(2-hydroxy-ethyl)benzamide, 2-[3-(3-bromo-5-chlorophenyl)ureido]-4-trifluoromethoxy-*N*-(2-hydroxy-ethyl)benzamide;
**7. R1** is **-CH₂CH₂CH₂OH** and
   **R2-H:** 2-[3-(3-fluorophenyl)ureido]-*N*-(3-hydroxy-propyl)benzamide, 2-[3-(3-chlorophenyl)ureido]-*N*-(3-hydroxy-propyl)benzamide, 2-[3-(3-bromophenyl)ureido]-*N*-(3-hydroxy-propyl)benzamide, 2-[3-(3,5-difluorophenyl)ureido]-*N*-(3-hydroxy-propyl)benzamide, 2-[3-(3,5-dichlorophenyl)ureido]-*N*-(3-hydroxy-propyl)benzamide, 2-[3 -(3,5-dibromophenyl)ureido]-*N*-(3-hydroxy-propyl)benzamide, 2-[3-(3-chloro-5-fluorophenyl)ureido]-*N*-(3-hydroxy-propyl)benzamide, 2-[3-(3-bromo-5-chlorophenyl)ureido]-*N*-(3-hydroxypropyl)benzamide,
   **R2-F:** 2-[3 -(3 -fluorophenyl)ureido]-4-fluoro-*N*-(3-hydroxy-propyl)benzamide, 2-[3 -(3 - chlorophenyl)ureido]-4-fluoro-*N*-(3-hydroxy-propyl)benzamide, 2-[3-(3-bromophenyl)ureido]-4-fluoro-*N*-(3-hydroxy-propyl)benzamide, 2-[3-(3,5-difluorophenyl)ureido]-4-fluoro-*N*-(3-hydroxy-propyl)benzamide, 2-[3-(3,5-dichlorophenyl)ureido]-4-fluoro-*N*-(3-hydroxy-propyl)benzamide, 2-[3-(3,5-dibromophenyl)ureido]-4-fluoro-*N*-(3-hydroxy-propyl)benzamide, 2-[3-(3-chloro-5-fluorophenyl)ureido]-4-fluoro-*N*-(3-hydroxy-propyl)benzamide, 2-[3-(3-bromo-5-chlorophenyl)ureido]-4-fluoro-*N*-(3-hydroxy-propyl)benzamide,
   **R2-Cl:** 4-chloro-2-[3-(3-fluorophenyl)ureido]-*N*-(3-hydroxy-propyl)benzamide, 4-chloro-2-[3-(3-chlorophenyl)ureido]-*N*-(3-hydroxy-propyl)benzamide, 4-chloro-2-[3-(3-bromophenyl)ureido]-*N*-(3-hydroxy-propyl)benzamide, 4-chloro-2-[3-(3,5-difluorophenyl)ureido]-*N*-(3-hydroxy-propyl)benzamide, 4-chloro-2-[3-(3,5-dichlorophenyl)ureido]-*N*-(3-hydroxy-propyl)benzamide, 4-chloro-2-[3-(3,5-dibromophenyl)ureido-*N*-(3-hydroxy-propyl)benzamide, 2-[3-(3-chloro-5-fluorophenyl)ureido]-4-chloro-*N*-(3-hydroxy-propyl)benzamide, 2-[3-(3-bromo-5-chlorophenyl)ureido]-4-chloro-*N*-(3-hydroxy-propyl)benzamide,
   **R2-Br:** 4-bromo-2-[3-(3-fluorophenyl)ureido]-*N*-(3-hydroxy-propyl)benzamide, 4-bromo-2-[3-(3-chlorophenyl)ureido]-*N*-(3-hydroxy-propyl)benzamide, 4-bromo-2-[3-(3-bromophenyl)ureido]-*N*-(3-hydroxy-propyl)benzamide, 4-brom-2-[3-(3,5-difluorophenyl)ureido]-*N*-(3-hydroxy-propyl)benzamide, 4-bromo-2-[3-(3,5-dichlorophenyl)ureido]-*N*-(3-hydroxy-propyl)benzamide, 4-bromo-2-[3-(3,5-dibromophenyl)ureido]-*N*-(3-hydroxy-propyl)benzamide, 4-bromo-2-[3-(3-chloro-5-fluorophenyl)ureido] -*N*-(3-hydroxy-propyl)benzamide, 2-[3-(3-bromo-5-chlorophenyl)ureido]-4-bromo-*N*-(3-hydroxy-propyl)benzamide,
   **R2-OCH₃:** 2-[3-(3-fluorophenyl)ureido]-4-methoxy-*N*-(3-hydroxy-propyl)benzamide, 2-[3 -(3 - chlorophenyl)ureido]-4-methoxy-*N*-(3-hydroxy-propyl)benzamide, 2-[3-(3-bromophenyl)ureido]-4-methoxy-*N-*(3-hydroxy-propyl)benzamide, 2-[3-(3,5-difluorophenyl)ureido]-4-methoxy-*N*-(3-hydroxy-propyl)benzamide, 2-[3-(3,5-dichlorophenyl)ureido]-4-methoxy-*N*-(3-hydroxy-propyl)benzamide, 2-[3-(3,5-dibromophenyl)ureido]-4-methoxy-*N*-(3-hydroxy-propyl)benzamide, 2-[3-(3-chloro-5-fluorophenyl)ureido]-4-methoxy-*N*-(3-hydroxy-propyl)benzamide, 2-[3-(3-bromo-5-chlorophenyl)ureido]-4-methoxy-*N*-(3-hydroxy-propyl)benzamide,
   **R2-OCF₃**: 2-[3-(3-fluorophenyl)ureido]-4-trifluoromethoxy-*N*-(3-hydroxy-propyl)benzamide, 2-[3-(3-chlorophenyl)ureido]-4-trifluoromethoxy-*N*-(3-hydroxy-propyl)benzamide, 2-[3-(3-bromophenyl)ureido]-4-trifluoromethoxy-*N*-(3-hydroxy-propyl)benzamide, 2-[3-(3,5-difluorophenyl)ureido]-4-trifluoromethoxy-*N*-(3-hydroxy-propyl)benzamide, 2-[3-(3,5-dichlorophenyl)ureido]-4-trifluoromethoxy-*N*-(3-hydroxy-propyl)benzamide, 2-[3-(3,5-dibromophenyl)ureido]-4-trifluoromethoxy-*N*-(3-hydroxy-propyl)benzamide, 2-[3-(3-chloro-5-fluorophenyl)ureido]-4-trifluoromethoxy-*N*-(3-hydroxy-propyl)benzamide, 2-[3-(3-bromo-5-chlorophenyl)ureido]-4-trifluoromethoxy-*N*-(3-hydroxy-propyl)benzamide;
**8. R1** is **CH₂CH₂NH₂** and
   **R2-H:** 2-[3-(3-fluorophenyl)ureido]-*N*-(2-amino-ethyl)benzamide, 2-[3-(3-chlorophenyl)ureido]-*N*-(2-amino-ethyl)benzamide, 2-[3-(3-bromophenyl)ureido]-*N*-(2-amino-ethyl)benzamide, 2-[3-(3,5-difluorophenyl)ureido]-*N*-(2-amino-ethyl)benzamide, 2-[3-(3,5-dichlorophenyl)ureido]-*N-*(2-amino-ethyl)benzamide, 2-[3-(3,5-dibromophenyl)ureido]-*N*-(2-amino-ethyl)benzamide, 2-[3-(3-chloro-5-fluorophenyl)ureido]-*N*-(2-amino-ethyl)benzamide, 2-[3-(3-bromo-5-chlorophenyl)ureido]-*N*-(2-amino-ethyl)benzamide,
   **R2-F:** 2-[3-(3-fluorophenyl)ureido]-4-fluoro-*N*-(2-amino-ethyl)benzamide, 2-[3-(3-chlorophenyl)ureido]-4-fluoro-*N*-(2-amino-ethyl)benzamide, 2-[3-(3-bromophenyl)ureido]-4-fluoro-*N*-(2-amino-ethyl)benzamide, 2-[3-(3,5-difluorophenyl)ureido]-4-fluoro-*N*-(2-aminoethyl)benzamide, 2-[3-(3,5-dichlorophenyl)ureido]-4-fluoro-*N*-(2-amino-ethyl)benzamide, 2-[3-(3,5-dibromophenyl)ureido]-4-fluoro-*N*-(2-amino-ethyl)benzamide, 2-[3-(3-chloro-5-fluorophenyl)ureido]-4-fluoro-*N*-(2-amino-ethyl)benzamide, 2-[3-(3-bromo-5-chlorophenyl)ureido]-4-fluoro-*N*-(2-amino-ethyl)benzamide,
   **R2-Cl:** 4-chloro-2-[3-(3-fluorophenyl)ureido]-*N*-(2-amino-ethyl)benzamide, 4-chloro-2-[3-(3-chlorophenyl)ureido]-*N*-(2-amino-ethyl)benzamide, 4-chloro-2-[3-(3-bromophenyl)ureido]-*N*-(2-amino-ethyl)benzamide, 4-chloro-2-[3-(3,5-difluorophenyl)ureido]-*N*-(2-aminoethyl)benzamide, 4-chloro-2-[3-(3,5-dichlorophenyl)ureido]-*N*-(2-amino-ethyl)benzamide, 4-chloro-2-[3-(3,5-dibromophenyl)ureido]-*N*-(2-amino-ethyl)benzamide, 2-[3-(3-chloro-5-fluorophenyl)ureido]-4-chloro-*N*-(2-amino-ethyl)benzamide, 2-[3-(3-bromo-5-chlorophenyl)ureido]-4-chloro-*N*-(2-amino-ethyl)benzamide,
   **R2-Br:** 4-bromo-2-[3-(3-fluorophenyl)ureido]-*N*-(2-amino-ethyl)benzamide, 4-bromo-2-[3-(3-chlorophenyl)ureido]-*N*-(2-amino-ethyl)benzamide, 4-bromo-2-[3-(3-bromophenyl)ureido]-*N*-(2-amino-ethyl)benzamide, 4-brom-2-[3-(3,5-difluorophenyl)ureido]-*N*-(2-amino-ethyl)benzamide, 4-bromo-2-[3-(3,5-dichlorophenyl)ureido]-*N*-(2-amino-ethyl)benzamide, 4-bromo-2-[3-(3,5-dibromophenyl)ureido]-*N*-(2-amino-ethyl)benzamide, 4-bromo-2-[3-(3-chloro-5-fluorophenyl)ureido] -*N*-(2-amino-ethyl)benzamide, 2-[3-(3-bromo-5-chlorophenyl)ureido]-4-bromo-*N*-(2-amino-ethyl)benzamide,
   **R2-OCH₃:** 2-[3-(3-fluorophenyl)ureido]-4-methoxy-*N*-(2-amino-ethyl)benzamide, 2-[3-(3-chlorophenyl)ureido]-4-methoxy-*N-*(2-amino-ethyl)benzamide, 2-[3-(3-bromophenyl)ureido]-4-methoxy-*N*-(2-amino-ethyl)benzamide, 2-[3-(3,5-difluorophenyl)ureido]-4-methoxy-*N*-(2-amino-ethyl)benzamide, 2-[3-(3,5-dichlorophenyl)ureido]-4-methoxy-*N*-(2-amino-ethyl)benzamide, 2-[3-(3,5-dibromophenyl)ureido]-4-methoxy-*N*-(2-amino-ethyl)benzamide, 2-[3-(3-chloro-5-fluorophenyl)ureido]-4-methoxy-*N*-(2-amino-ethyl)benzamide, 2-[3-(3-bromo-5-chlorophenyl)ureido]-4-methoxy-*N*-(2-amino-ethyl)benzamide,
   **R2-OCF₃**: 2-[3-(3-fluorophenyl)ureido]-4-trifluoromethoxy-*N*-(2-amino-ethyl)benzamide, 2-[3-(3-chlorophenyl)ureido]-4-trifluoromethoxy-*N*-(2-amino-ethyl)benzamide, 2-[3-(3-bromophenyl)ureido]-4-trifluoromethoxy-*N*-(2-amino-ethyl)benzamide, 2-[3-(3,5-difluorophenyl)ureido]-4-trifluoromethoxy-*N*-(2-amino-ethyl)benzamide, 2-[3-(3,5-dichlorophenyl)ureido]-4-trifluoromethoxy-*N*-(2-amino-ethyl)benzamide, 2-[3-(3,5-dibromophenyl)ureido]-4-trifluoro-methoxy-*N*-(2-amino-ethyl)benzamide, 2-[3-(3-chloro-5-fluorophenyl)ureido]-4-trifluorometh-oxy-*N*-(2-amino-ethyl)benzamide, 2-[3-(3-bromo-5-chlorophenyl)ureido]-4-trifluoromethoxy-*N*-(2-amino-ethyl)benzamide.

In the most preferred embodiment, the compound of the general formula (I) is selected from the group comprising:
2-[3-(3-fluorophenyl)ureido]benzamide, 2-[3-(3,5-difluorophenyl)ureido]benzamide, 2-[3-(3,5-dichlorophenyl)ureido]benzamide, 2-[3-(3-chloro-5-fluorophenyl)ureido]benzamide, 2-[3-(3-fluorophenyl)ureido]-4-methoxybenzamide, 2-[3-(3-chlorophenyl)ureido]-4-methoxybenzamide, 2-[3-(3,5-difluorophenyl)ureido]-4-methoxybenzamide, 2-[3-(3,5-dichlorophenyl)ureido]-4-methoxybenzamide, 2-[3-(3-chloro-5-fluorophenyl)ureido]-4-methoxybenzamide, 2-[3-(3-fluorophenyl)ureido]-4-trifluoromethoxybenzamide, 2-[3-(3-chlorophenyl)ureido]-4-trifluoromethoxybenzamide, 2-[3-(3,5-difluorophenyl)ureido]-4-trifluoromethoxybenzamide, 2-[3-(3,5-dichlorophenyl)ureido]-4-trifluoromethoxybenzamide, 2-[3-(3-chloro-5-fluorophenyl)ureido]-4-trifluoromethoxybenzamide.

Further object of the present invention is the use of the compound of the general formula (I) in agriculture, in particular for increasing crop yields and increasing their resistance to stress, such as drought, heat, cold, frost and salinity.

The compounds of the general formula (I) of the present invention exhibits the highest known CKX inhibitory activity. As a result, they increase the cytokinin levels in plants, which leads to their higher viability and higher yield.

The compound of general formula (I) may be used for delaying chlorophyll degradation and plant tissue senescence. This will also increase the viability of the plants in stress conditions and the yield of economically important parts of plants, such as seeds, fruits, tubers and others. This unique effect is due to the effect of the compound of the general formula (I) on increasing endogenous cytokinin levels in plants.

The compounds of the general formula (I) of the present invention for use in agriculture, can be applied to whole plants, plant organs, or to plant cells and tissues. The compounds of the general formula (I) according to the present invention are also suitable for use in plant tissue cultures, where they prevent the degradation of cytokinins and thus increase the viability of new plants, regeneration of buds and shoots from explants, callus growth, suppress apical dominance, reduce hyperhydricity of plants grown *in vitro* and show very strong micropropagation activity.

The compounds of the general formula (I) may be used as inhibitors of stress and stress-induced senescence in crop production, in particular cereals (wheat, barley, rice, maize, rye, oats, sorghum and related species), beet (sugar beet and fodder beet); malvaceae, stone fruit and soft fruits (apples, pears, plums, peaches, almonds, cherries, strawberries and blackberries); legumes (beans, lentils, peas, soybeans); oilseeds (rape, mustard, poppy, olives, sunflower, coconut, ricinus, cocoa beans, groundnuts); cucumbers (pumpkins, cucumbers, melons); fibrous plants (cotton, flax, hemp, jute); citrus fruits (oranges, lemons, grapefruits, tangerines); vegetables (spinach, cinnamon, camphor) or plants such as tobacco, nuts, eggplant, sugar cane, tea, vines, hops, bananas and natural rubber and medicinal plants, as well as ornamental plants. Stress can be caused by drought, heat and salinity (saline stress).

The object of the present invention further includes antisenescent and/or antistress preparations for plants, plant organs and plant cells, comprising at least one compound of the general formula (I) and at least one auxiliary substance (excipient). The auxiliary substances can be in solid or liquid state.

Excipients refer to solvents, solid carriers, surface active agents (surfactants), emulsifiers, dispersants, wetting agents, stabilizers, defoamers, preservatives, viscosity agents, adhesives, and also fertilizers or other active ingredients.

Suitable solvents are, for example, aromatic hydrocarbons, preferably C₈-C₁₂ aromatic hydrocarbons (e.g. xylene or naphthalene derivatives), phthalates (for example dibutyl phthalate, dioctyl phthalate), aliphatic hydrocarbons (cyclohexane, paraffin), alcohols, glycols and their ethers and esters (ethanol, ethylene glycol, 2- methoxyethanol, 2-ethoxyethanol), ketones (cyclohexanone, N-methyl-2-pyrrolidone, DMSO, DMF), vegetable oils, water. Solid carriers are typically calcite, talc, kaolin, montmorillonite or attapulgite, silicic acid, polymers, crushed limestone. Examples of suitable anionic, non-ionic and cationic surfactants are listed, for example, in WO 97/34485. Stabilizers are, for example, vegetable oils or epoxidized vegetable oils (epoxidized palm oil, rapeseed or olive oil). The defoamer is, for example, silicone oil.

The compounds of the general formula (I) are used in unmodified form or, preferably, together with excipients conventionally employed in the art of preparations. To this end they are conveniently formulated as concentrates of active compounds as well as suspensions and dispersions, preferentially isotonic water solutions, suspensions and dispersion, diluted emulsions, soluble powders, dusts, granulates, creams, gels, oil suspensions and also encapsulations, e.g. polymeric substances. As with the type of the preparation, the methods of application, such as spraying, atomizing, dusting, scattering, coating or pouring, are chosen in accordance with the intended 5 objectives and the prevailing circumstances. The preparations may be sterilized and/or contain further excipients of neutral nature such as preservatives, stabilizers, wetting agents or emulgators, solubilizing agents, as well as fertilizers, micronutrient donors or other formulations for obtaining special effects.

The invention further relates to a method for inhibiting stress and/or senescence in plants, plant organs and/or plant cells, which comprises applying at least one compound of the general formula (I) to a plant, plant organ and/or plant cell.

The compounds of this invention can be prepared by chemical reactions known in the prior art (Scheme 1). The process for the preparation of the compounds of the general formula (I) is based on the reaction of 1 equivalent of substituted isocyanatobenzene with 1 equivalent of (substituted) 2-aminobenzoic acid (Step B). Preferably, isocyanatobenzene can be prepared from a aniline derivative by a conventional method using diphosgene (Step A, Kurita K. et al, 1976, J. Org. Chem. 41, 2070-71). The obtained isocyanate (or commercially available isocyanate) is then reacted with (substituted) 2-aminobenzoic acid to give a 2-(3-phenylureido)benzoic acid derivative (Step B), which can be converted to a 2-(3-phenylureido)derivative of benzamide (Step C) by any of the known reactions.

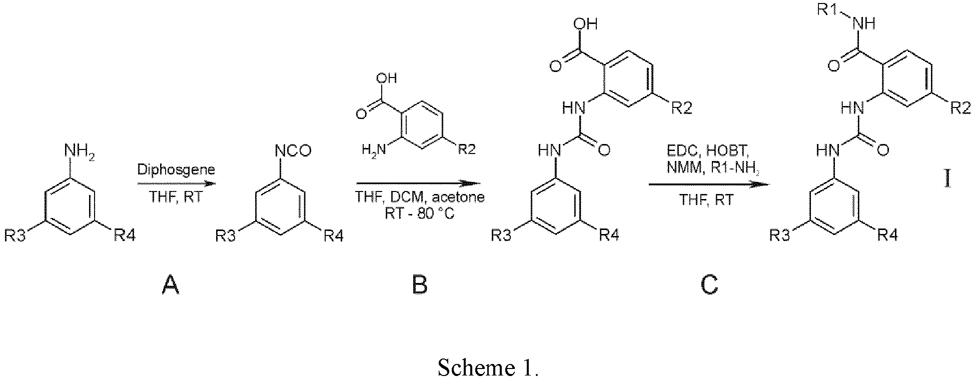

### PREPARATIONS

The preparations comprising the compounds of general formula I (active ingredients) and, where appropriate, one or more solid or liquid excipients, are prepared in a manner known per se e.g. by mixing and/or grinding the active ingredients with excipients, e.g. solvents or solid carriers. In addition, surface-active compounds (surfactants) may also be used in the preparations. Depending on the nature of the compound of general formula (I) to be formulated, suitable surface-active compounds are non-ionic, cationic and/or anionic surfactants and surfactant mixtures having good emulsifying, dispersing and wetting properties.

Examples of suitable anionic, non-ionic and cationic surfactants are listed, for example, in WO 97/34485.

Also suitable in the preparation of the compositions containg compounds of the general formula (I) according to the invention are the surfactants conventionally used in formulation technology, which are described, *inter alia,* in "McCutcheon's Detergents and Emulsifiers Annual" MC Publishing Corp., Ridgewood New Jersey, 1981; Stache, H., "Tensid-Taschenbuch", Carl Hanser Verlag, Munich, 1981; and M. and J. Ash, "Encyclopedia of Surfactants", Vol.l-3, Chemical Publishing Co., New York, 1980-81.

The formulation of the preparation contains from 0.01 to 99.09 % (w/w), preferably from 0.1 to 95 % (w/w) of the compound of general formula (I) as the active ingredient, and from 99.09 to 0.01% (w/w) of the excipient defined above, preferably from 5 to 99.9 % of a mixture of additives or other carriers, depending on the methods of application, and optionally containing from 0.1 up to 25 % (w/w) of wetting agent.

Whereas commercial products are usually formulated as concentrates, the end user will normally employ dilute formulations. The compositions may also comprise further ingredients, such as stabilisers, e.g. vegetable oils or epoxidised vegetable oils (epoxidised coconut 0;1, rapeseed oil or soybean oil), antifoams, e.g. silicone oil, preservatives, stabilizers, wetting agents or emulsifiers, viscosity factors, binders, tackifiers, and also fertilisers or other active ingredients. Preferred formulations have especially the following compositions: (% = percent by weight)

### Emulsifiable concentrates:

active ingredient mixture: 5 to 90 %, surfactant: 1 to 30 %, liquid carrier: 5 to 94 %

### Dusts:

active ingredient mixture: 0.1 to 10 %, solid carrier: 99.9 to 90 %,

### Suspension concentrates:

active ingredient mixture: 0.5 to 75 %, water: 94 to 24 %, surfactant: 1 to 40 %,

### Wettable powders:

active ingredient mixture: 0.5 to 90 %, surfactant: 0.5 to 20 %, solid carrier: 5 to 95 %,

### Granules:

active ingredient mixture: 0.1 to 30 %, solid carrier: 99.9 to 70 %.

The compositions may also comprise further ingredients, such as stabilisers, e.g. vegetable oils or epoxidised vegetable oils (epoxidised coconut oil, rapeseed oil or soybean oil), anti-foams, e.g. silicone oil, preservatives, viscosity regulators, binders, tackifiers, and also fertilisers or other active ingredients. For the use of the compounds of general formula (I), or of compositions comprising them, in the protection of crop plants against the damaging effects of growth regulators, various methods and techniques come into consideration, such as, for example, the following:
i) Seed dressing
   a) Dressing of the seeds with a wettable powder formulation of a compound of the general formula (I) by shaking in a vessel until uniformly distributed over the seed surface (dry dressing). In that procedure approximately from 1 to 500 g of compound of the general formula (I) (4 g to 2 kg of wettable powder) are used per 100 kg of seed.
   b) Dressing of the seeds with an emulsifiable concentrate of a compound of formula (I) according to method a) (wet dressing).
   c) Dressing by immersing the seeds for from 1 to 72 hours in a liquor comprising from 100 to 1000 ppm of a compound of general formula (I) and preferably subsequently drying the seeds (immersion dressing).
   Dressing the seeds or treating the germinated seedlings are naturally the preferred methods of application, because treatment with the active ingredients is directed entirely at the target crop. Generally from 1 to 1000 g, preferably from 5 to 250 g of the compound of general formula (I), are used per 100 kg of seed, but depending on the methodology, which also enables the addition of other active ingredients or micronutrients, the concentration limits indicated can be varied up or down (repeat dressing).
ii) Application as a tank mixture
   A liquid formulation is used in the amount of 0.005 to 5.0 kg per hectare. Such tank mixtures are applied before or after sowing.
iii) Application to the seed furrow
   The compounds of formula (I) are introduced into the open sown seed furrow in the form of an emulsifiable concentrate, wettable powder or granules. Once the seed furrow has been covered over, the growth regulator is applied in the usual manner in the pre-emergence process.
iv) Controlled release of active ingredient
   The compounds of formula (I) are applied in solution to mineral granule carriers or polymerised granules (urea/formaldehyde) and dried. If desired, it is also possible to apply a coating that allows the active ingredient to be released in metered amounts over a specific period of time (coated granules).

### Examples

All compounds that were synthesized and belonging to the scope of the general formula (I) were prepared by reacting 1 equivalent of substituted isocyanatobenzene with 1 equivalent of (substituted) 2-aminobenzoic acid according to Scheme 1 (step B). The urea derivative with a carboxyl group was then converted to a urea derivative with an amide or *N*-substituted amide group according to Scheme 1 (step C). Commercially available isocyanates used in the present invention: 3-fluorophenylisocyanate, 3-chlorophenylisocyanate, 3-bromophenylisocyanate, 3,5-difluorophenylisocyanate, 1,3-dichloro-5-isocyanato-benzene, 3,5-dibromophenylisocyanate. If the isocyanate is not commercially available, it was prepared by the known reaction of a substituted aromatic amine with diphosgene in THF (Kurita K. et al., 1976, J. Org. Chem. 41, 2070-71) according to Scheme 1 (step A). The isocyanate prepared by this method was used in crude form in further syntheses (Scheme 1, step B). All aromatic amines for the preparation of isocyanates are commercially available.

### Example 1. Preparation of isocyanates

One equivalent of substituted aniline was dissolved in dry THF (10 mL) and mixed with two equivalents of triethylamine. This mixture was slowly added dropwise to a solution of diphosgene (0.6 equivalent) in THF (10 mL) at -20 °C. The temperature of the reaction mixture (RM) was then brought to room teperature (r.t.) or higher (about 60 °C). The precipitated triethylamine hydrochloride was filtered off and the filtrate was evaporated to dryness. The crude isocyanate remained in the reaction vessel and was used for the next synthesis. The following isocyanates were prepared by this method: 1-Chloro-3-isocyanato-5-(trifluoromethoxy)benzene, 1-chloro-3-fluoro-5-isocyanatobenzene, 1-bromo-3-chloro-5-isocyanatobenzene. These isocyanates and isocyanates purchased were used to synthesize all other compounds.

### Example 2. 2-[3-(3,5-Dichlorophenyl)ureido]benzamide (6)

1,3-Dichloro-5-isocyanatobenzen (84 mg, 0.5 mmol) was dissolved in acetone and 2-aminobenzamide (68 mg, 0.5 mmol) was added with stirring. The RM was stirred at 55 °C overnight. The product crystallized from DCM. Yield 97 mg (60 %). **EM** 323. **HPLC:** purity 99.7 %, *R*ₜ = 25.8 min. **¹H NMR** (500 MHz, DMSO-*d₆*): δ 7.01 (1H, t, *J*=7.7 Hz, ArH), 7.12 (1H, t, *J*=1.8 Hz, ArH), 7.42 (1H, t, *J*=7.7 Hz, ArH), 7.56 (2H, d, *J*=1.8 Hz, ArH), 7.67 (1H, s (br), CONH₂), 7.70 (1H, d, *J*=8.0 Hz, ArH), 8.19-8.23 (2H, d, *J*=8.0 Hz, ArH + 1H, s (br), CONH₂), 10.06 (1H, s, NH-urea), 10.81 (1H, s, NH-urea). **MS-ESI⁺** coin voltage (CV) 20 V, *m*/*z* (rel. int.): 346.0 [M+Na]⁺ (100), 348.0 [M+Na]⁺ (100). **MS-ESI⁻** CV 20 V*, m*/*z* (rel. int.): 321.8 [M-H]⁻(100), 323.7 [M-H]⁻ (70). **UV** (MeOH), λ (nm): 214 (maxi), 238 (min₁), 265 (max₂), 288 (min₂), 305 (maxs).

### Example 3. 2-[3-(3-Chloro-5-fluorophenyl)ureido]benzamide (8)

1-Chloro-3-fluoro-5-isocyanatobenzen (86 mg, 0.5 mmol) was dissolved in DCM and 2-aminobenzamide (68 mg, 0.5 mmol) was added. The RM was stirred at 55 °C overnight. The product crystallized from methanol. White crystal, yield 95 mg (62 %). **EM** 307. **HPLC:** purity 98.6 %, *R*ₜ = 25.2 min. **¹H NMR** (500 MHz, DMSO-*d₆*): 6.95 (1H, td, *J_{d}=8.6* Hz, *Jₜ*=1.7 Hz, ArH), 7.02 (1H, t, *J*=7.7 Hz, ArH), 7.34 (1H, td, *J_{d}*=11.5 Hz, *Jₜ*=1.7 Hz, ArH), 7.39-7.45 (2H, m, ArH), 7.66 (1H, s(br), CONH₂), 7.70 (1H, d, J=6.9 Hz, ArH), 8.18-8.23 (2H, d+s(br), *J_{d}*=8.0 Hz, ArH and CONH₂), 10.07 (1H, s, NH), 10.79 (1H, s, NH). **MS-ESI⁺** CV 30 V, *m*/*z* (rel. int.): 330.0 and 332.0 [M+Na]⁺ (100 and 30). **MS-ESI⁻** CV 15 V, *m*/*z* (rel. int.): 306.0 and 308.0 [M-H]⁻ (100 and 30), 342.0 [M+Cl]⁻ (40), 352.0 [M+HCOOH]⁻ (50).

### Example 4. 2-[3-(3-Chloro-5-trifluoromethoxyphenyl)ureido]benzamide (11)

1-Chlor-3-isocyanato-5-trifluoromethoxybenzen (118 mg, 0.5 mmol) was dissolved in aceton and 2-aminobenzamide (68 mg, 0.5 mmol) was added. The RM was stirred at 70 °C for 5 hours. The product crystallized from DCM. White crystal, yield 120 mg (64 %). **EM** 373. **HPLC:** purity 98.3 %, *R*ₜ = 26.2 min. **¹H NMR** (500 MHz, DMSO-*d₆*): δ 7.02 (1H, t, *J*=7.3 Hz, ArH), 7.07 (1H, s, ArH), 7.43 (1H, t, *J*=7.3 Hz, ArH), 7.54 (1H, s, ArH), 7.61 (1H, s, ArH), 7.67 (1H, s (br), CONH₂), 7.71 (1H, d, *J*=8.0 Hz, ArH), 8.21 (1H, d, *J*=8.0 Hz, ArH), 8.23 (1H, s (br), CONH₂), 10.17 (1H, s, NH), 10.86 (1H, s, NH). **MS-ESI⁺** CV 20 V, *m*/*z* (rel. int.): 395.8 and 397.8 [M+Na]⁺ (100 and 40). **MS-ESI⁻** CV 20 V, *m*/*z* (rel. int.): 371.8 [M-H]⁻ (100), 373.6 [M-H]⁻ (40). **UV** (MeOH), λ (nm): 212 (maxi), 235 (min), 264 (max₂), 309 (sh).

### Example 5. Synthesis of amides from carboxylic acids

Some compounds containing an amide group or an *N*-substituted amide group were prepared from carboxylic acids by a method using 1-ethyl-3-(3-dimethylaminopropyl)-carbodiimide hydrochloride (EDC), hydroxybenzotriazole (HOBt) and *N*-methylmorpholine (NMM) (Schema 1, step C). In detail: The benzoic acid derivative (1.0 equivalent) is dissolved in THF, then EDC (1.1 equivalent), HOBt (1.0 equivalent) and NMM (1.1 equivalent) are added sequentially. The RM is stirred for 30 minutes at room temperature and then an excess of ammonia solution or another aliphatic amine or amino alcohol (in THF, water or other solvent) is added. The RM is stirred overnight at room temperature. After the reaction, the THF is evaporated and the residue is partitioned between water and ethyl acetate. In the organic layer is the desired product, which after evaporation usually gave a pure crystalline product.

### Example 6. 4-Chloro-2-[3-(3,5-dichlorophenyl)ureido]benzamide (16)

1,3-Dichloro-5-isocyanatobenzen (94 mg, 0.5 mmol) was dissolved in aceton and 2-amino-4-chloro-benzoic acid (86 mg, 0.5 mmol) was added with stirring. The RM was stirred at 55 °C overnight. The product crystallized from DCM. 4-Chloro-2-[3-(3,5-dichlorophenyl)ureido] benzoic acid was filterred off and dried. Yield 155 mg (86 %). **EM** 358. **¹H NMR** (500 MHz, DMSO-*d₆*): δ 7.00 (1H, dd, *J₁*=8.6 Hz, *J₂*=2.3Hz, ArH), 7.12 (1H, t, *J*=1.9 Hz, ArH), 7.60 (2H, d, *J*=2.3 Hz, ArH), 7.94 (1H, d, *J*=8.6 Hz, ArH), 8.38 (1H, d, *J*=2.3 Hz, ArH), 10.20 (1H, s, NH), 12.06 (1H, s, NH). The final amide was obtained according to Example 5 using NH₄OH solution (aq). Yield 141 mg (91 %). **EM** 357. **¹H NMR** (500 MHz, DMSO-*d₆*): δ 7.10 (1H, dd, *J₁*=8.6 Hz, *J₂*=2.3 Hz, ArH), 7.15 (1H, t, *J*=1.7 Hz, ArH), 7.55 (2H, d, *J*=1.7 Hz, ArH), 7.73 (1H, d, *J*=8.6 Hz, ArH), 7.76 (1H, s (br), CONH₂), 8.29 (1H, s (br), CONH₂), 8.37 (1H, d, *J*=2.3 Hz, ArH), 10.18 (1H, s, NH), 11.03 (1H, s, NH). **MS-ESI⁺** CV 20 V, *m*/*z* (rel. int.): 359.9 [M+H]⁺ (40), 379.8 [M+Na]⁺ (100), 738.7 [2M+Na]⁺ (40). **MS-ESI⁻** CV 20 V, *m*/*z* (rel. int.): 355.8 [M-H]⁻(100), 358.0 [M-H]⁻ (70), 359.3 [M-H]⁻ (20).

### Example 7. 4-Bromo-2-[3-(3,5-dichlorophenyl)ureido]benzamide (19)

1,3-Dichloro-5-isocyanatobenzen (188 mg, 1 mmol) was dissolved in aceton and 2-amino-4-bromobenzoic acid (216,4 mg, 1 mmol) was added. The RM was stirred at 55 °C overnight. The product precipitated from water. White crystals of 4-bromo-2-[3-(3,5-dichlorophenyl)ureido]-benzoic acid was filtered off, dried and then washed with DCM. Yield 380 mg (94 %). **EM** 403. **MS-ESI⁻** CV 15 V, *m*/*z* (rel. int.): 400.8, 402.7 and 404.7 [M-H]⁻ (50, 100 and 50). The final amide was obtained according to Example 5 using NH₄OH solution (aq). Starting material was 0.12 mmol (50 mg), isolated was 45 mg, (90 %). **EM** 402. **¹H NMR** (500 MHz, DMSO-*d₆*): δ 7.14 (1H, t (br), ArH), 7.23 (1H, dd, *J₁*=8.6 Hz, *J₂*=1.7 Hz, ArH), 7.55 (2H, d, *J*=1.7 Hz, ArH), 7.66 (1H, d, *J*=8.6 Hz, ArH), 7.77 (1H, s (br), CONH₂), 8.29 (1H, s (br), CONH₂), 8.51 (1H, d, *J*=1.7 Hz, ArH), 10.18 (1H, s, NH), 10.98 (1H, s, NH). **MS-ESI⁺** CV 20 V, *m*/*z* (rel. int.): 400.8 [M+H]⁺ (30), 402.8 [M+H] ⁺ (50), 423.8 [M+Na]⁺ (70), 425.8 [M+Na]⁺ (100), 427.7 [M+Na]⁺ (45). **MS-ESI⁻** CV 20 V, *m*/*z* (rel. int.): 399.7 [M-H]⁻(45), 401.7 [M-H]⁻(100), 403.8 [M-H]⁻ (40).

### Example 8. 2-[3-(3,5-Dichlorophenyl)ureido]-4-methoxybenzamide (24)

1,3-Dichloro-5-isocyanatobenzen (94 mg, 0.5 mmol) was dissolved in aceton and 2-amino-4-methoxybenzoic acid (84 mg, 0.5 mmol) was added. The RM was stirred at r.t. overnight. The product crystallized from DCM. White crystals of 2-[3-(3,5-dichlorophenyl)ureido]-4-methoxybenzoic acid were filtered off and dried. Yield 160 mg (90 %). The final amide was obtained according to Example 5 using NH₄OH solution (aq). White crstals, yield 145 mg, 90 %. **EM** 353. **¹H NMR** (500 MHz, DMSO-*d₆*): δ 3.76 (3H, s, OCH₃), 6.57 (1H, d, *J*=8.0 Hz, ArH), 7.12 (1H, s (br), ArH), 7.47 (1H, s (br), CONH₂), 7.57 (2H, s, ArH), 7.71 (1H, d, *J*=8.6 Hz, ArH), 7.94 (1H, s, ArH), 8.07 (1H, s (br), CONH₂), 10.66 (1H, s, NH), 11.43 (1H, s, NH). **MS-ESI⁻** CV 20 V, *m*/*z* (rel. int.): 351.8 [M-H]⁻(100), 353.7 [M-H]⁻ (75).

### Example 9. 2-[3-(3,5-Dichlorophenyl)ureido]-4-trifluoromethoxybenzamide (28)

1,3-Dichloro-5-isocyanatobenzen (94 mg, 0.5 mmol) was dissolved in aceton and 2-amino-4-trifluoromethoxybenzamide (110 mg, 0.5 mmol) was added. The latter was obtained from commercially available 2-amino-4-trifluoromethoxy-benzonitrile by partial basic hydrolysis (in ethanol and water (1:1) with 4 equivalents of NaOH, room temperature, overnight). **EM** 220, **MS-ESI⁺** CV 15 V, *m*/*z* (rel. int.): 204.0 [M-NH₃]⁺ (100), 221.0 [M+H]⁺ (60). **MS-ESI⁻** CV 20 V, *m*/*z* (rel. int.): 218.9 [M-H]⁻ (100). The RM was stirred at r.t. overnight. The product crystallized from chloroform. White crystals, yield 157 mg, 77 %. **EM** 407. **¹H NMR** (500 MHz, DMSO-*d₆*): δ 7.01 (1H, d, *J*=8.5 Hz, ArH), 7.15 (1H, t, *J*=2.3 Hz, ArH), 7.55 (2H, d, *J*=2.3 Hz, ArH), 7.80 (1H, s, ArH), 7.84 (1H, d, *J*=8.5 Hz, ArH), 8.32 (2H, s, CONH₂), 10.26 (1H, s, NH), 11.05 (1H, s, NH). **MS-ESI⁺** CV 25 V, *m*/*z* (rel. int.): 429.9 and 431.9 [M+Na]⁺ (100 and 70). **MS-ESI⁻** CV 25 V, *m*/*z* (rel. int.): 405.8 and 307.8 [M-H]⁻ (100 and 70).

### Example 10. 4-Bromo-2-[3-(3,5-dichlorophenyl)ureido]-N-ethylbenzamide (35)

4-Bromo-2-[3-(3,5-dichlorophenyl)ureido]-benzoic acid prepared according to Example 7 (60 mg, 0.15 mmol) was dissolved in THF and step by step EDC, HOBt, NMM and freshly prepared solution of THF and water (1:0.3) containing ethylamine hydrobromide (1.5 mmol) and triethylamine (1.5 mmol) were added as described in Example 5. The pure product was obtained by column chromatography (20% methanol in chloroform). Yield 20 mg, 33 %. **EM** 430. **¹H NMR** (500 MHz, DMSO-*d₆*): δ 1.06 (3H, t, *J*=7.5 Hz, CH₃), 3.27 (2H, m, *J*=5.8 Hz, CH₂), 7.15 (1H, t, *J*=1.5 Hz, ArH), 7.25 (1H, dd, *J₁*=8.5 Hz, *J₂*=2.0 Hz, ArH), 7.55 (2H, d, *J*=2.0 Hz, ArH), 7.60 (1H, d, *J*=8.6 Hz, ArH), 8.5 (1H, d, *J*=2.0 Hz, ArH), 8.80 (1H, t, *J*=5.7 Hz, CONH), 10.19 (1H, s, NH), 10.78 (1H, s, NH). **MS-ESI⁺** CV 20 V, *m*/*z* (rel. int.): 430.0 [M+H]⁺ (75), 431.9 [M+H]⁺ (100), 433.9 [M+H]⁺ (70). **MS-ESI⁻** CV 20 V, *m*/*z* (rel. int.): 427.8 [M-H]⁻ (50), 429.8 [M-H]⁻(100), 431.7 [M-H]⁻ (40).

### Example 11. 1-(5-Bromo-2-hydrazinocarbonylphenyl)-3-(3,5-dichlorophenyl)-urea (45)

4-Bromo-2-[3-(3,5-dichlorophenyl)ureido]-benzoic acid prepared according to Example 7 (50 mg, 0.12 mmol) was dissolved in THF and EDC, HOBt, NMM and hydrazin (3.0 mmol) was added. The RM was stirred at r.t. overnight. The crude product was washed with alkaline water solution. Yield 30 mg, 60 %. **EM** 417. **¹H NMR** (500 MHz, DMSO-*d₆*): δ 4.62 (2H, s (br), NH**NH₂**), 7.14 (1H, t, *J*=1.7 Hz, ArH), 7.23 (1H, dd, *J₁*=8.6 Hz, *J₂*=1.7 Hz, ArH), 7.52 (1H, d, *J*=8.6 Hz, ArH), 7.54 (2H, d, *J*=1.7 Hz, ArH), 8.45 (1H, d, *J*=1.7 Hz, ArH), 10.01 (1H, s (br), CONHNH₂), 10.22 (1H, s, NH-urea), 10.53 (1H, s (br), NH-urea). **MS-ESI⁺** CV 20 V, *m*/*z* (rel. int.): 416.8 [M+H]⁺ (50), 418.8 [M+H]⁺ (100), 440.8 [M+Na]⁺ (80), 443.0 [M+Na]⁺ (45). **MS-ESI⁻** CV 20 V, *m*/*z* (rel. int.): 414.4 [M-H]⁻(45), 416.6 [M-H]⁻(100), 418.3 [M-H]⁻ (45).

### Example 12. 4-Bromo-2-[3-(3,5-dichlorophenyl)ureido]-N-(2-hydroxy-ethyl)benzamide (48)

4-Bromo-2-[3-(3,5-dichlorophenyl)ureido]-benzoic acid prepared according to Example 7 (60 mg, 0.15 mmol) was dissolved in THF and EDC, HOBt, NMM and etanolamin (1.5 mmol) was added. The RM was stirred at r.t. overnight. Yield 55 mg, 92 %. **EM** 446. **¹H NMR** (500 MHz, DMSO-*d₆*): δ 3.38 (2H, q, *J*=4.7 Hz, NH**CH₂**), 3.51 (2H, q, *J*=4.7 Hz, **CH₂**OH), 7.76 (1H, t (br), OH), 7.14 (1H, t, *J*=1.7 Hz, ArH), 7.25 (1H, dd, *J₁*=8.6 Hz, *J₂*=2.3 Hz, ArH), 7.55 (2H, d, *J*=1.7 Hz, ArH), 7.64 (1H, d, *J*=8.6 Hz, ArH), 8.50 (1H, d, *J*=2.3 Hz, ArH), 8.77 (1H, t (br), CONH), 10.19 (1H, s, NH), 10.74 (1H, s (br), NH). **MS-ESI⁺** CV 20 V, *m*/*z* (rel. int.): 446.0, 447.9 and 450.0 [M+H]⁺ (70, 100 and 65). **MS-ESI⁻** CV 20 V, *m*/*z* (rel. int.): 327.8 [M-H]⁻ (50), 445.9 and 447.9 [M-H]⁻(100, 40).

### Example 13. N-(2-Aminoethyl)-4-bromo-2-[3-(3,5-dichlorophenyl)ureido]benzamide (55)

4-Bromo-2-[3-(3,5-dichlorophenyl)ureido]-benzoic acid prepared according to Example 7 (50 mg, 0.12 mmol) was dissolved in THF and EDC, HOBt, NMM and 1,2-diaminoetan (1 mL) was added. The RM was stirred at r.t. overnight. The final product crystallized from chloroform. Yield 44 mg, 88 %. **EM** 445. **¹H NMR** (500 MHz, DMSO-*d₆*): δ 2.67 (2H, t, *J*=6.8 Hz, CH₂), 3.25 (2H, t, *J*=6.3 Hz, CH₂), 7.14 (1H, t, *J*=1.8 Hz, ArH), 7.25 (1H, dd, *J₁*=8.0 Hz, *J₂*=2.0 Hz, ArH), 7.55 (2H, d, *J*=1.8 Hz, ArH), 7.64 (1H, d, *J*=8.6 Hz, ArH), 8.48 (1H, d, *J*=2.0 Hz, ArH), 8.76 (1H, s (br), NH), 10.17 (1H, s (br), NH). **MS-ESI⁺** CV 12 V, *m*/*z* (rel. int.): 444.9, 446.9 and 448.9 [M+H]⁺ (60, 100 and 50). **MS-ESI⁻** CV 20 V, *m*/*z* (rel. int.): 442.6, 444.8 and 446.5 [M-H]⁻ (40, 100 and 30).

Additional compounds listed in Table 1. were prepared following the procedures in Examples 1-13.

**Table 1. Overview of substituents in positions R1-R4 for compounds synthesized, their MS-ESI values [M+H]⁺ and IC₅₀ values in units of micromoles with enzyme ZmCKX1.**

| Explanations: D - di, F - fluorine, Cl - chlorine, Br - bromine, Me - methyl, OMe - methoxy group, TFM - trifluoromethoxy group, AD - amido group, MAD - *N*-methylamido group, EAD - *N-*ethylamido group, PAD - *N*-propylamido group, HD - hydrazido group, HED - *N*-(2-hydroxyethyl)amido group, HPD - *N*-(3-hydroxypropyl)amido group, AED - *N*-(2-aminoethyl)amido group. CPPU is 1-(2-chloro-4-pyridyl)-3-phenylurea, DCPPU is 1-(2,6-dichloropyridin-4-yl)-3-phenylurea, F-INCYDE is 2-fluoro-9*H*-purine-6-yl)-(3-methoxyphenyl)amine, 3,5DCl-Ph-U is 1-(3,5-dichlorophenyl)-3-phenylurea. The number indicates the position of the substituent on the phenyl ring in relation to urea bridge, a dash separates the two phenyl rings. Abbreviations begin with a ring bearing an amide group. Abbreviations do not contain letters for the words phenyl and urea (except for the standard - 3,5DCl-Ph-U). | | | | | | | |
|---|---|---|---|---|---|---|---|
| **c. n.** | **compound abbreviation** | **R1** | **R2** | **R3** | **R4** | **MS-ESI [M+H]** | ***IC₅₀* (µM) ZmCKX1** |
| | CPPU | standard | | | | | 18 |
| | DCPPU | standard | | | | | 4.5 |
| | F-INCYDE | standard | | | | | 1 |
| | 3,5DCl-Ph-U | standard | | | | | 0.84 |
| **1** | 2AD-3F | H | H | F | H | 274.2 | 0.450 |
| **2** | 2AD-3Br | H | H | Br | H | 335.1 | 0.340 |
| **3** | 2AD-3OMe | H | H | OCH₃ | H | 286.3 | 0.320 |
| **4** | 2AD-3TFM | H | H | OCF₃ | H | 340.1 | 0.210 |
| **5** | 2AD-3,5DF | H | H | F | F | 292.0 | 0.160 |
| **6** | 2AD-3,5DCl | H | H | Cl | Cl | 324.1 | 0.022 |
| **7** | 2AD-3,5DBr | H | H | Br | Br | 414.3 | 0.047 |
| **8** | 2AD-3C1,5F | H | H | F | Cl | 308.1 | 0.110 |
| **9** | 2AD-3Br,5Cl | H | H | Br | Cl | 367.0 | 0.040 |
| **10** | 2AD-3Cl,5Me | H | H | Cl | CH₃ | 304.1 | 0.070 |
| **11** | 2AD-3C1,5TFM | H | H | Cl | OCF₃ | 375.3 | 0.120 |
| **12** | 2AD,5F-3Cl | H | F | Cl | H | 309.2 | 0.090 |
| **13** | 2AD,5F-3,5DCl | H | F | Cl | Cl | 342.3 | 0.018 |
| **14** | 2AD,5F-3Cl,5OMe | H | F | Cl | OCH₃ | 338.1 | 0.028 |
| **15** | 2AD,5 Cl-3 Cl | H | Cl | Cl | H | 324.0 | 0.035 |
| **16** | 2AD,5Cl-3,5DCl | H | Cl | Cl | Cl | 358.2 | 0.002 |
| **17** | 2AD,5Cl-3Br,5Cl | H | Cl | Br | Cl | 403.3 | 0.001 |
| **18** | 2AD,5Br-3Cl | H | Br | Cl | H | 367.1 | 0.011 |
| **19** | 2AD,5Br-3,5DCl | H | Br | Cl | Cl | 403.0 | 0.001 |
| **20** | 2AD,5Br-3Cl,5OMe | H | Br | Cl | OCH₃ | 399.1 | 0.034 |
| **21** | 2AD,5OMe-3F | H | OCH₃ | F | H | 304.2 | 0.058 |
| **22** | 2AD,5OMe-3Cl | H | OCH₃ | Cl | H | 320.3 | 0.035 |
| **23** | 2AD,5OMe-3Br | H | OCH₃ | Br | H | 365.3 | 0.033 |
| **24** | 2AD,5OMe-3,5DCl | H | OCH₃ | Cl | Cl | 354.1 | 0.001 |
| **25** | 2AD,5OMe-3Br,5Cl | H | OCH₃ | Br | Cl | 399.4 | 0.001 |
| **26** | 2AD,5TFM-3Cl | H | OCF₃ | Cl | H | 374.3 | 0.009 |
| **27** | 2AD,5TFM-3Br | H | OCF₃ | Br | H | 419.2 | 0.007 |
| **28** | 2AD,5TFM-3,5DCl | H | OCF₃ | Cl | Cl | 407.1 | 0.001 |
| **29** | 2MAD-3,5DCl | CH₃ | H | Cl | Cl | 338.0 | 0.030 |
| **30** | 2MAD,5F-3,5DCl | CH₃ | F | Cl | Cl | 356.2 | 0.021 |
| **31** | 2MAD,5Cl-3,5DCl | CH₃ | Cl | Cl | Cl | 372.3 | 0.014 |
| **32** | 2MAD,5Br-3,5DCl | CH₃ | Br | Cl | Cl | 417.2 | 0.010 |
| **33** | 2MAD,5OMe-3Cl | CH₃ | OCH₃ | Cl | H | 334.1 | 0.006 |
| **34** | 2EAD-3,5DCl | CH₂CH₃ | H | Cl | Cl | 352.1 | 0.038 |
| **35** | 2EAD,5Br-3,5DCl | CH₂CH₃ | Br | Cl | Cl | 431.2 | 0.003 |
| **36** | 2EAD,5OMe-3Cl | CH₂CH₃ | OCH₃ | Cl | H | 348.2 | 0.021 |
| **37** | 2EAD,5OMe-3,5DCl | CH₂CH₃ | OCH₃ | Cl | H | 382.3 | 0.002 |
| **38** | 2EAD,5TFM-3Cl,5F | CH₂CH₃ | OCF₃ | Cl | F | 420.1 | 0.001 |
| **39** | 2PAD-3F | CH₂CH₂CH₃ | H | F | H | 316.2 | 0.17 |
| **40** | 2PAD-3,5DF | CH₂CH₂CH₃ | H | F | F | 334.1 | 0.062 |
| **41** | 2PAD-3,5DCl | CH₂CH₂CH₃ | H | Cl | Cl | 366.0 | 0.030 |
| **42** | 2PAD,5Cl-3,5DF | CH₂CH₂CH₃ | Cl | F | F | 367.1 | 0.022 |
| **43** | 2PAD,5OMe-3,5DCl | CH₂CH₂CH₃ | OCH₃ | Cl | Cl | 396.1 | 0.004 |
| **44** | 2HD-3,5DCl | NH₂ | H | Cl | Cl | 339.2 | 0.074 |
| **45** | 2HD,5Br-3,5DCl | NH₂ | Br | Cl | Cl | 418.1 | 0.020 |
| **46** | 2HD,5OMe-3,5DCl | NH₂ | OCH₃ | Cl | Cl | 369.3 | 0.018 |
| **47** | 2HED-3,5DCl | CH₂CH₂OH | H | Cl | Cl | 368.3 | 0.022 |
| **48** | 2HED,5Br-3,5DCl | CH₂CH₂OH | Br | Cl | Cl | 447.1 | 0.005 |
| **49** | 2HED,5OMe-3F | CH₂CH₂OH | OCH₃ | F | H | 348.2 | 0.040 |
| **50** | 2HED,5OMe-3,5DCl | CH₂CH₂OH | OCH₃ | Cl | Cl | 398.1 | 0.003 |
| **51** | 2HPD-3Cl | CH₂CH₂CH₂OH | H | Cl | H | 348.3 | 0.150 |
| **52** | 2HPD,5Br-3Cl | CH₂CH₂CH₂OH | Br | Cl | H | 427.1 | 0.080 |
| **53** | 2HPD,5OMe-3,5DCl | CH₂CH₂CH₂OH | OCH₃ | Cl | Cl | 412.1 | 0.020 |
| **54** | 2AED,5Br-3Cl | CH₂CH₂NH₂ | Br | Cl | H | 412.0 | 0.035 |
| **55** | 2AED,5Br-3,5DCl | CH₂CH₂NH₂ | Br | Cl | Cl | 446.2 | 0.010 |
| **56** | 2AED,5OMe-3,5DCl | CH₂CH₂NH₂ | OCH₃ | Cl | Cl | 397.1 | 0.013 |
| **57** | 2AED,5TFM-3,5DCl | CH₂CH₂NH₂ | OCF₃ | Cl | Cl | 451.2 | 0.008 |

### Example 14. Compounds inhibit the activity of the maize enzyme cytokinin oxidase/dehydrogenase 1 (ZmCKX1)

The *IC₅₀* (concentration of a substance that causes 50% inhibition of the enzyme activity) was measured by PMS/MTT assay [phenazine methosulphate/3-(4,5-dimethylthiazol-2-yl)-2,5-diphenyltetrazolium bromide]. The assay was performed in 96-well microtiter plates. Each well contained a reaction mixture with a final concentration of: 0.1 M KH₂PO₄, pH 7.4, 1 mM MTT, 0.2 mM PMS, tested substance in a concentration range of 0.1 nM - 1 µM and 10 µM N⁶-isopentenyladenine (iP) as a substrate. To this reaction mixture 100 µL of medium containing the active enzyme ZmCKX1 was added. The enzyme was produced by the yeast *Yarrowia lipolytica* and isolated according to the protocol (Kopečný D. et al., 2005, Biochemistry 87:1011-1022). The plates were incubated for 30 min at 37 °C and the enzymatic reaction was stopped by the addition of 25 µL of 35% acetic acid. Then, the absorbance at 578 nm was measured on a spectrophotometer, and the absorbance of the sample without the substrate was substracted as a reference value. The *IC₅₀* value was determined from the concentration-dependent curves in GraphPad Prism software. The values shown in Table 1 are the average of at least two measurements each performed in three repetitions. All of the compounds tested have significantly lower *IC₅₀* values than the known CKX inhibitors CPPU, DCPPU and 3,5DCl-Ph-U. For comparison, we also present the *IC₅₀* value for the known CKX inhibitor F-INCYDE (Zatloukal M. et al., 2008, Bioorganic & Medicinal Chemistry 16, 9268-9275), which is, however, a phenyl-adenine derivative. The substance 3,5DCl-Ph-U is 1-(3,5-dichlorophenyl)-3-phenylurea, which we have been prepared to compare its inhibitory activity with the compounds presented in the present invention. Addition of the amide group to the unsubstituted phenyl ring of this substance gives the compound 2AD-3,5DC, which has 42-fold better inhibitory activity with the ZmCKX1 enzyme than 3,5DCl-Ph-U. F-INCYDE ((2-fluoro-9H-purin-6-yl)-(3-methoxyphenyl)amine), CPPU (1-(2-chloropyridin-4-yl)-3-phenylurea), and DCPPU (1-(2,6-dichloropyridin-4-yl)-3-phenylurea) are substances known in the prior art. All these compounds are less effective than the new generation of urea derivatives of the present invention.

### Example 15. Foliar application of compound 24 increases the seed yield in Arabidopsis

It has previously been shown that a reduction of CKX activity in Arabidopsis has led to an increase in the number of flowers/pods and an increase in the number of seeds in each silique. The result was a 55 % increase in total seed yield compared to control plants (Bartrina et al., 2011, Plant Cell 23, 69-80).

In our experiment, young *Arabidopsis thaliana* plants were grown in small pots in soil and they were sprayed with a 0.01 µM compound **24** (aq solution) in phase of 4 true leaves (about 14-20 days after sowing). Control plants were sprayed with an aqueous solution of DMSO (c = 0.01% (v/v)) and silwet (c = 0.001% (v/v)). Both treated (n = 25) and control plants (n = 25) were further grown under optimal conditions until their natural death. Subsequently, seeds from all plants were harvested and the yield was evaluated (Table 2). While control plants had an average of 41 (± 8) seeds per silique and a total seed yield per plant of 122 (± 16) mg, treated plants had an average of 50 (± 5) seeds per silique and a total seed yield per plant was 148 (± 17) mg. This means an increase in total seed yield of 21 %.

The whole experiment was repeated with a similar result: control plants had an average of 43 (± 6) seeds per silique and a total seed yield per plant of 115 (± 12) mg, treated plants had an average of 52 (± 7) seeds per one silique and the total seed yield per plant was 135 (± 13) mg. This means an increase in total seed yield of 17 %.

**Table 2. Foliar application of 24 increases seed yield of Arabidopsis thaliana**

| | Yield (mg) | Yield (%) | benefit |
|---|---|---|---|
| Control | 122 ± 16 | 100 % | |
| 0.01 µM compound **24** | 148 ± 17 | **121 %** | **+ 21 %** |
| Kontrola | 115 ± 12 | 100 % | |
| 0.01 µM compound **24** | 135 ± 13 | **117 %** | **+ 17 %** |

### Example 16. Foliar application with compound 24 prolongs the life span of Arabidopsis thaliana in drought stress conditions and reduces the yield losses

Young *Arabidopsis thaliana* plants grown in small pots in soil were sprayed with a 0.01 µM aqueous solution of substance **24** in phase of four true leaves (within 14-20 days after sowing). Control plants were sprayed with an aqueous solution of DMSO (c = 0.01% (v/v)) and silwet (c = 0.001% (v/v)). Control plants were further divided into watered plants and those with reduced watering (drought stressed). The plants treated with substance **24** had also reduced water regime, the same as part of the control plants. All plants were grown until the stage of their natural death. Control watered plants lived for an average of 85 days before dying spontaneously. Their seed yield in average was 141 (± 22) mg. Control plants with reduced watering lived only 61 days on average and the average yield was 95 (± 14) mg. Plants treated with substance **24** and with reduced watering lived for an average of 75 days and their average seed yield was 117 (± 16) mg. These data clearly show (Table 3) that CKX inhibitors significantly increase the yield and viability of Arabidopsis plants that have been exposed to environmental stress.

**Table 3. Foliar application with compound 24 reduces Arabidopsis thaliana seed yield losses**

| | Yield (mg) | Yield (%) | benefit |
|---|---|---|---|
| Watered control | 141 ± 22 | 100 % | |
| Cotrol with reduced waterring | 95 ± 14 | 67 % | |
| Plants with reduced waterring + 0.01 µM compound **24** | 117 ± 16 | 83 % | **+ 16 %** |

### Example 17. Application of substance 24 increases the seed yield of barley in field conditions

The effect of compound **24** on seed yield of barley and its phenotype was investigated in a field trials. All field experiments were performed according to Good agricultural practices, ie. six areas of 10 m² were used for one variant. Furthermore, the crop was not treated with any other growth regulator (substance **24** only) or fungicide. Compound **24** was applied to seeds at a concentration of 10 µM before sowing. In 2016 barley seeds treated by this way were grown in the field and the yield of these plants was compared with the untreated control (Table 4). The treated plants had a seed yield of 105.3 % compared to the control (100 %). Phenotype analysis further showed that the higher yield could be due to the higher number of tillers which the treated plants had 14 % more than the control plants.

In 2017, compound **24** was applied to barley leaves at a concentration of 5 µM (in a volume of 200 L per hectare) in two different growth phases - BBCH 21-23 and BBCH 51. Both applications increased the seed yield to 106.3 % and 106, 8 % versus control (100 %), respectively (Table 4).

**Table 4. Application of substance 24 increases the seed yield of barley in field conditions**

| | Growth phase | Yield (%) | benefit |
|---|---|---|---|
| Control | | 100 % | |
| 10 µM compound **24** (seed application) | | **105.3 %** | **+ 5.3 %** |
| 5 µM compound **24** (foliar application) | BBCH 21-23 | **106.3 %** | **+ 6.3 %** |
| 5 µM compound **24** (foliar application) | BBCH 51 | **106.8 %** | **+ 6.8 %** |

### Example 18. Application of substance 24 increases the seed yield of winter rapeseed in field conditions

In 2018, the effect of foliar application of substance **24** (concentration 1 µM in a volume of 300 L per hectare) on the yield of winter rapeseed was investigated in field conditions. The field trials were again performed according to Good Agricultural Practice. Substance **24** was applied to plants in two different growth phases - BBCH 30 and BBCH 33-35. In the first case, the rapeseed yield increased to 106.2 % compared to the control, and in the second case, the seed yield increased to 108.8 % compared to the control (100 %) (Table 5). Furthermore, the seeds of the treated plants were found to be slightly heavier, the weight of one thousand seeds in the control was 5.38 g. The weight of one thousand seeds in the treated plants was 5.6 g (BBCH 30) and 5.8 g (BBCH 33-35). This phenomenon also contributed to the increase in total seed yield.

**Table 5. Application of substance 24 increases the seed yield of winter rapeseed in field conditions**

| | growth phase | yield (%) | benefit |
|---|---|---|---|
| Control | | 100% | |
| 1 µM compound **24** (foliar application) | BBCH 30 | **106.2 %** | **+ 6.2 %** |
| 1 µM compound **24** (foliar application) | BBCH 33-35 | **108.8 %** | **+ 8.8 %** |

**Example 19.** The application of compound **24** increases the seed yield of wheat in field conditions In 2017, the effect of the application of substance **24** on wheat yield in field conditions was investigated. The substance was again applied either to wheat seeds before sowing (= seed dressing with substance **24** at a concentration of 50 µM) or to leaves (= spraying with substance **24** at a concentration of 5 µM) in two different growth phases - BBCH 25 and BBCH 51. All field experiments were performed according to Good Agricultural Practice. In the case of seed dressing, the yield was increased to 104.0 % compared to the control (100 %). In the case of foliar application, the yield was increased to 105.9 % (BBCH 21) and 105.0 % (BBCH 51) compared to the control (100 %) (Table 6). Phenotype analysis showed that the treated plants had about 7 % more tillers than the control plants.

**Table 6. Application of compound 24 increases seed yield of wheat in field conditions**

| | growth phase | yield (%) | benefit |
|---|---|---|---|
| Control | | 100% | |
| 50 µM compound **24** (seedapplication) | | **104.0 %** | **+ 4.0 %** |
| 5 µM compound **24** (foliar application) | BBCH 25 | **105.9 %** | **+ 5.9 %** |
| 5 µM compound **24** (foliar application) | BBCH 51 | **105.0 %** | **+ 5.0 %** |

### Example 20. Formulations

The active ingredient in the formulations may be any compound of formula (I) or mixture of two or more compounds of general formula (I).

| 41. Emulsifiable concentrates | a) | b) | c) | d) |
|---|---|---|---|---|
| active ingredient mixture | 5 % | 10 % | 25 % | 50 % |
| calcium dodecylbenzenesulfonate | 6 % | 8 % | 6 % | 8 % |
| castor oil polyglycol ether (36 mol of ethylene oxide) | 4 % | - | 4 % | 4 % |
| octylphenol polyglycol ether (7-8 mol of ethylene oxide) | 2 % | - | 2 % | - |
| cyclohexanone | - | - | 10 % | 20 % |
| arom. hydrocarbon mixture C₉-C₁₂ | 83 % | 82 % | 53 % | 18 % |

Emulsions of any desired concentration can be obtained from such concentrates by dilution with water.

| A2. Solutions | a) | b) | c) | d) |
|---|---|---|---|---|
| active ingredient mixture | 5 % | 10 % | 50 % | 90 % |
| 1-methoxy-3-(3-methoxy-propoxy)-propane | - | 20 % | 20 % | - |
| polyethylene glycol MW 400 | 20 % | 10 % | - | - |
| N-methyl-2-pyrrolidone | - | - | 30 % | 10 % |
| arom. hydrocarbon mixture C₉-C₁₂ | 75 % | 60 % | - | - |

The solutions are suitable for use in the form of microdrops.

| A3. Wettable powders | a) | b) | c) | d) |
|---|---|---|---|---|
| active ingredient mixture | 5 % | 25 % | 50 % | 80 % |
| sodium lignosulfonate | 4 % | - | 3 % | - |
| sodium lauryl sulfate s | 2 % | 3 % | - | 4 % |
| odium diisobutylnaphthalene-sulfonate | - | 6 % | 5 % | 6 % |
| octylphenol polyglycol ether (7-8 mol of ethylene oxide) | 1 % | 2 % | - | - |
| highly dispersed silicic acid | 1 % | 3 % | 5 % | 10 % |
| kaolin | 87 % | 61 % | 37 % | - |

The active ingredient is mixed thoroughly with the adjuvants and the mixture is thoroughly ground in a suitable mill, affording wettable powders which can be diluted with water to give suspensions of any desired concentration.

| A4. Coated granules | a) | b) | c) |
|---|---|---|---|
| active ingredient mixture | 0.1 % | 5 % | 15 % |
| highly dispersed silicic acid | 0.9 % | 2 % | 2 % |
| inorganic carrier (.AE 0.1 -1 mm) | 99.0 % | 93 % | 83 % |
| e.g. CaCO₃ or SiO₂ | | | |

The active ingredient is dissolved in methylene chloroide and applied to the carrier by spraying, and the solvent is then evaporated off in vacuo.

| A5. Coated granules | a) | b) | c) |
|---|---|---|---|
| active ingredient mixture | 0.1 % | 5 % | 15 % |
| polyethylene glycol MW 200 | 1.0 % | 2 % | 3 % |
| highly dispersed silicic acid | 0.9 % | 1 % | 2 % |
| inorganic carrier (AE 0.1 -1 mm) | 98.0 % | 92 % | 80 % |
| e.g. CaCO₃ or SiO₂ | | | |

The finely ground active ingredient is uniformly applied, in a mixer, to the carrier moistened with polyethylene glycol. Non-dusty coated granules are obtained in this manner.

| A6. Extruder granules | a) | b) | c) | d) |
|---|---|---|---|---|
| active ingredient mixture | 0.1 % | 3 % | 5 % | 15 % |
| sodium lignosulfonate | 1.5 % | 2 % | 3 % | 4 % |
| carboxymethylcellulose | 1.4 % | 2 % | 2 % | 2 % |
| kaolin | 97 % | 93 % | 90 % | 79 % |

The active ingredient is mixed and ground with the adjuvants, and the mixture is moistened with water. The mixture is extruded and then dried in a stream of air.

| A7. Dusts | a) | b) | c) |
|---|---|---|---|
| active ingredient mixture | 0.1 % | 1 % | 5 % |
| talcum | 39.9 % | 49 % | 35 % |
| kaolin | 60 % | 50 % | 60 % |

Ready-to-use dusts are obtained by mixing the active ingredient with the carriers and grinding the mixture in a suitable mill.

| A8. Suspension concentrates | a) | b) | c) | d) |
|---|---|---|---|---|
| active ingredient mixture | 3 % | 10 % | 25 % | 50 % |
| ethylene glycol | 5 % | 5 % | 5 % | 5 % |
| nonylphenol polyglycol ether (15 mol of ethylene oxide) | 1 % | 2 % | - | - |
| sodium lignosulfonate | 3 % | 3 % | 4 % | 5 % |
| carboxymethylcellulose | 1 % | 1 % | 1 % | 1 % |
| 37 % aqueous formaldehyde solution | 0.2 % | 0.2 % | 0.2 % | 0.2 % |
| silicone oil emulsion | 0.8 % | 0.8 % | 0.8 % | 0.8 % |
| water | 86 % | 78 % | 64 % | 38 % |

The finely ground active ingredient is intimately mixed with the adjutants, giving a suspension concentrate from which suspensions of any desired concentration can be obtained by dilution with water.

## Claims

1. A compound of the general formula (I), 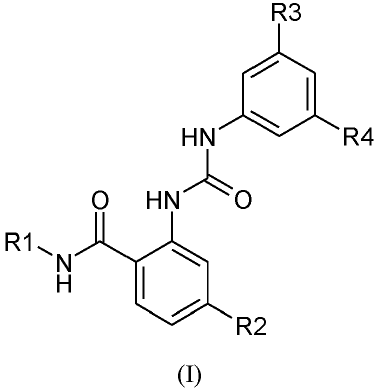 wherein
R1 is selected from the group consisting of hydrogen, amino group, C₁-C₅ alkyl, C₂-C₅ alkenyl, C₂-C₅ alkynyl, wherein C₁-C₅ alkyl can optionally be substituted with hydroxy and/or amino group;
R2 is selected from the group consisting of hydrogen, halogen, methoxy group and halogenated methoxy group;
R3 is selected from the group consisting of hydrogen, halogen, methyl, methoxy group and trifluoromethoxy group;
R4 is selcted from the group consisting of halogen, methyl, methoxy group and trifluoromethoxy group;
with the proviso that 2-[3-(3-chlorophenyl)ureido]benzamide is excluded.

2. The compound of the general formula (I) according to claim 1, wherein R1 is selected from the group consisting of hydrogen, methyl, ethyl, propyl, -NH₂, 2-hydoxyethyl, 3-hydoxypropyl and 2-aminoethyl.

3. The compound of the general formula (I) according to claim 1 or 2, wherein R2 is selected from the group consisting of H, F, Cl, Br, -OCH₃ and -OCF₃.

4. The compound of the general formula (I) according to claim 1, 2 or 3, wherein at least one of R3 and R4 is halogen, preferably both R3 and R4 are independently halogenes, selected from the group consisting of F, Cl and Br.

5. The compound of the general formula (I) according to claim 1, 2 or 3, wherein one of R3 and R4 is halogen, selected from the group consisting of F, Cl and Br, and at the same time the other one of R3 and R4 is selected from the group consisting of H, -CH₃, -OCH₃ and -OCF₃.

6. The compound according to any one of the preceding claims 1 to 5, selected from the group consisting of: 2-[3-(3-fluorophenyl)ureido]benzamide, 2-[3-(3,5-difluorophenyl)ureido]benzamide, 2-[3-(3,5-dichlorophenyl)ureido]benzamide, 2-[3-(3-chloro-5-fluorophenyl)ureido]benzamide, 2-[3-(3-fluorophenyl)ureido]-4-methoxybenzamide, 2-[3-(3-chlorophenyl)ureido]-4-methoxybenzamide, 2-[3-(3,5-difluorophenyl)ureido]-4-methoxybenzamide, 2-[3-(3,5-dichlorophenyl)ureido]-4-methoxybenzamide, 2-[3-(3-chloro-5-fluorophenyl)ureido]-4-methoxybenzamide, 2-[3-(3-fluorophenyl)ureido]-4-trifluoromethoxybenzamide, 2-[3-(3-chlorophenyl)ureido]-4-trifluoromethoxybenzamide, 2-[3-(3,5-difluorophenyl)ureido]-4-trifluoromethoxybenzamide, 2-[3-(3,5-dichlorophenyl)ureido]-4-trifluoromethoxybenzamide, 2-[3-(3-chloro-5-fluorophenyl)ureido]-4-trifluoromethoxybenzamide.

7. A preparation for the treatment of plants, **characterized in that** it contains at least one compound of the general formula (I) according to any one of the preceding claims or a salt thereof with an alkali metal, ammonia or an amine in the form of a racemate or an optically active isomer, or an acid addition salt thereof;
and at least one auxiliary substance selected from the group comprising solvents, solid carriers, surfactants, emulsifiers, dispersants, wetting agents, wetting agents, stabilizers, defoamers, preservatives, viscosities, binders, adhesives, and fertilizers.

8. The preparation according to claim 7, **characterized in that** it contains from 0.01 to 99.09 % (w/w) of the compound of the general formula (I) according to any one of the claims 1 to 6, and from 99.09 to 0.01 % (w/w) of at least one auxiliary substance, preferably it contains from 0.1 to 95 % (w/w) the compound of the general formula (I) and from 5 to 99.9 % of the at least one auxiliary substance.

9. The preparation according to claim 7 or 8, **characterized in that** it is in the form of a solution, preferably an isotonic aqueous solution; suspension; dispersion; emulsion; powder; dust; granulate; cream; gel; oily suspension or coated formulation.

10. Use of the compound of the general formula (I) according to any one of the preceding claims 1 to 6 and/or of the preparation according to any one of the preceding claims 7 to 9 as an inhibitor of cytokinin oxidase in the production of crop plants.

11. Use of the compound of the general formula (I) according to any one of the preceding claims 1 to 6 and/or of the preparation according to any one of the preceding claims 7 to 9 for inhibiting stress in whole plants, plant organs, plant tissues and/or plant cells.

12. Use of the compound of the general formula (I) according to any one of the preceding claims 1 to 6 and/or of the preparation according to any one of the preceding claims 7 to 9 for stimulation of seed germination, seedling growth and plant growth and development.

## Patentansprüche

1. Verbindung der allgemeinen Formel (I), 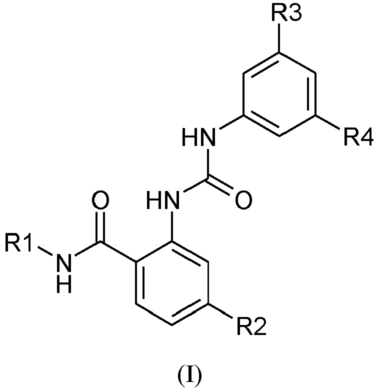 worin
R1 ist ausgewählt aus der Gruppe bestehend aus Wasserstoff, Aminogruppe, C₁-C₅-Alkyl, C₂-C₅-Alkenyl, C₂-C₅-Alkinyl, wobei C₁-C₅-Alkyl gegebenenfalls mit einer Hydroxy- und/oder Aminogruppe substituiert sein kann;
R2 ist ausgewählt aus der Gruppe bestehend aus Wasserstoff, Halogen, Methoxygruppe und halogenierten Methoxygruppe;
R3 ist ausgewählt aus der Gruppe bestehend aus Wasserstoff, Halogen, Methyl, Methoxygruppe und Trifluormethoxygruppe;
R4 ist ausgewählt aus der Gruppe bestehend aus Halogen, Methyl, Methoxygruppe und Trifluormethoxygruppe;
mit der Maßgabe, dass 2-[3-(3-Chlorphenyl)ureido]benzamid ausgeschlossen ist.

2. Verbindung der allgemeinen Formel (I) nach Anspruch 1, wobei R1 aus der Gruppe bestehend aus Wasserstoff, Methyl, Ethyl, Propyl, -NH₂, 2-Hydroxyethyl, 3-Hydroxypropyl und 2-Aminoethyl ausgewählt ist.

3. Verbindung der allgemeinen Formel (I) nach Anspruch 1 oder 2, wobei R2 aus der Gruppe bestehend aus H, F, Cl, Br, -OCH₃ und -OCF₃ ausgewählt ist.

4. Verbindung der allgemeinen Formel (I) nach Anspruch 1, 2 oder 3, wobei mindestens einer von R3 und R4 Halogen ist, vorzugsweise sind sowohl R3 als auch R4 unabhängig voneinander Halogene, ausgewählt aus der Gruppe bestehend aus F, Cl und Br.

5. Verbindung der allgemeinen Formel (I) nach Anspruch 1, 2 oder 3, wobei einer von R3 und R4 Halogen ist, ausgewählt aus der Gruppe bestehend aus F, Cl und Br, und gleichzeitig der andere von R3 und R4 sind ausgewählt aus der Gruppe bestehend aus H, -CH₃, -OCH₃ und -OCF₃.

6. Verbindung nach einem der vorhergehenden Ansprüche 1 bis 5, ausgewählt aus der Gruppe bestehend aus: 2-[3-(3-Fluorphenyl)ureido]benzamid, 2-[3-(3,5-Difluorphenyl)ureido]benzamid, 2-[3-(3,5-Dichlorphenyl)ureido]benzamid, 2-[3-(3-Chlor-5-fluorphenyl)ureido]benzamid, 2-[3-(3-Fluorphenyl)ureido]-4-methoxybenzamid, 2-[3-(3-Chlorphenyl)ureido]-4-methoxybenzamid, 2-[3-(3,5-Difluorphenyl)ureido]-4-methoxybenzamid, 2-[3-(3,5-Dichlorphenyl)ureido]-4-methoxybenzamid, 2-[3-(3-chlor-5-fluorphenyl)ureido]-4-methoxybenzamid, 2-[3-(3-Fluorphenyl)ureido]-4-trifluormethoxybenzamid, 2-[3-(3-Chlorphenyl)ureido]-4-trifluormethoxybenzamid, 2-[3-(3,5-Difluorphenyl)ureido]-4-trifluormethoxybenzamid, 2-[3-(3,5-Dichlorphenyl)ureido]-4-trifluormethoxybenzamid, 2-[3-(3-Chlor-5-fluorphenyl)ureido]-4-trifluormethoxybenzamid.

7. Präparat zur Behandlung von Pflanzen, **dadurch gekennzeichnet, dass** es mindestens eine Verbindung der allgemeinen Formel (I) nach einem der vorhergehenden Ansprüche oder ein Salz davon mit einem Alkalimetall, Ammoniak oder einem Amin in der Form eines Racemats oder eines optisch aktiven Isomers oder eines Säureadditionssalzes davon enthält;
und mindestens einen Hilfsstoff, ausgewählt aus der Gruppe bestehend aus Lösungsmitteln, festen Trägerstoffen, Tensiden, Emulgatoren, Dispergiermitteln, Netzmitteln, Netzmitteln, Stabilisatoren, Entschäumern, Konservierungsmitteln, Viskositätsmitteln, Bindemitteln, Klebstoffen und Düngemitteln.

8. Präparat nach Anspruch 7, **dadurch gekennzeichnet, dass** es 0,01 bis 99,09 % (Gew./Gew) der Verbindung der allgemeinen Formel (I) nach einem der Ansprüche 1 bis 6 enthält, und 99,09 bis 0,01 % (Gew./Gew.) mindestens eines Hilfsstoffs, vorzugsweise enthält es 0,1 bis 95 % (Gew./Gew.) der Verbindung der allgemeinen Formel (I) und 5 bis 99,9 % des mindestens einen Hilfsstoffs.

9. Präparat nach Anspruch 7 oder 8, **dadurch gekennzeichnet, dass** es in Form einer Lösung, vorzugsweise einer isotonischen wässrigen Lösung; Suspension; Dispersion; Emulsion; Pulver; Staub; granulieren; Creme; Gel; ölige Suspension oder beschichtete Formulierung vorliegt.

10. Verwendung der Verbindung der allgemeinen Formel (I) nach einem der vorhergehenden Ansprüche 1 bis 6 und/oder des Präparats nach einem der vorhergehenden Ansprüche 7 bis 9 als Inhibitor der Cytokininoxidase bei der Züchtung von Nutzpflanzen.

11. Verwendung der Verbindung der allgemeinen Formel (I) nach einem der vorhergehenden Ansprüche 1 bis 6 und/oder des Präparats nach einem der vorhergehenden Ansprüche 7 bis 9 zur Hemmung von Stress in ganzen Pflanzen, Pflanzenorganen, Pflanzengewebe und/oder Pflanzenzellen.

12. Verwendung der Verbindung der allgemeinen Formel (I) nach einem der vorhergehenden Ansprüche 1 bis 6 und/oder des Präparats nach einem der vorhergehenden Ansprüche 7 bis 9 zur Stimulierung der Samenkeimung, des Keimlingswachstums und Pflanzenwachstums und - entwicklung.

## Revendications

1. Un composé de formule générale (I), 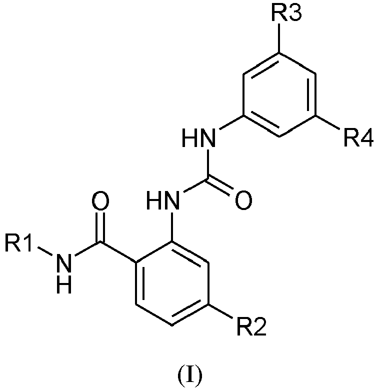 où
R1 est choisi dans le groupe constitué d'un hydrogène, d'un groupe amino, d'un (C₁-C₅)alkyle, d'un (C₂-C₅)alcényle, d'un (C₂-C₅)alcynyle, où un (C₁-C₅)alkyle peut éventuellement être substitué par un groupe hydroxy et/ou amino;
R2 est choisi dans le groupe constitué d'un hydrogène, d'un halogène, d'un groupe méthoxy et d'un groupe méthoxy halogéné;
R3 est choisi dans le groupe constitué d'un hydrogène, d'un halogène, d'un groupe méthyle, d'un groupe méthoxy et d'un groupe trifluorométhoxy;
R4 est choisi dans le groupe constitué d'un halogène, d'un groupe méthyle, d'un groupe méthoxy et d'un groupe trifluorométhoxy;
à condition que le 2-[3-(3-chlorophényl)uréido]benzamide soit exclu.

2. Le composé de formule générale (I) selon la revendication 1, dans lequel R1 est choisi dans le groupe constitué par l'hydrogène, le méthyle, l'éthyle, le propyle, -NH₂, 2-hydroxyéthyle, 3-hydroxypropyle et 2-aminoéthyle.

3. Le composé de formule générale (I) selon la revendication 1 ou 2, dans lequel R2 est choisi dans le groupe constitué de H, F, Cl, Br, -OCH₃ et -OCF₃.

4. Le composé de formule générale (I) selon la revendication 1, 2 ou 3, dans lequel au moins l'un de R3 et R4 est un halogène, de préférence R3 et R4 sont indépendamment des halogènes, choisis dans le groupe constitué de F, Cl et Br.

5. Le composé de formule générale (I) selon la revendication 1, 2 ou 3, dans lequel l'un de R3 et R4 est un halogène, choisi dans le groupe constitué de F, Cl et Br, et en même temps l'autre de R3 et R4 est choisi dans le groupe constitué de H, -CH₃, -OCH₃ et -OCF₃.

6. Le composé selon l'une quelconque des revendications 1 à 5 précédentes, choisi dans le groupe constitué de: 2-[3-(3-fluorophényl)uréido]benzamide, 2-[3-(3,5-difluorophényl)uréido]benzamide, 2-[3-(3,5-dichlorophényl)uréido]benzamide, 2-[3-(3-chloro-5-fluorophényl)uréido]benzamide, 2- [3 -(3 -fluorophényl)uréido] -4-méthoxybenzamide, 2-[3-(3-chlorophényl)uréido]-4-méthoxybenzamide, 2-[3-(3,5-difluorophényl)uréido]-4-méthoxybenzamide, 2-[3-(3,5-dichlorophényl)uréido]-4-méthoxybenzamide, 2-[3-(3-chloro-5-fluorophényl)uréido]-4-méthoxybenzamide, 2-[3-(3-fluorophényl)uréido]-4-trifluorométhoxybenzamide, 2-[ 3-(3-chlorophényl)uréido]-4-trifluorométhoxybenzamide, 2-[3-(3,5 -difluorophényl)uréido] -4-trifluorométhoxybenzamide, 2- [3 -(3,5 -dichlorophényl)uréido] -4-trifluorométhoxybenzamide, 2-[3-(3-chloro-5-fluorophényl)uréido]-4-trifluorométhoxybenzamide.

7. Une préparation pour le traitement des plantes, **caractérisée en ce qu'**elle contient au moins un composé de formule générale (I) selon l'une quelconque des revendications précédentes ou un sel de celui-ci avec un métal alcalin, de l'ammonium ou une amine sous forme d'un racémate ou d'un isomère optiquement actif, ou d'un sel d'addition acide de celui-ci;
et au moins une substance auxiliaire sélectionnée dans le groupe comprenant les solvants, les supports solides, les tensioactifs, les émulsifiants, les dispersants, les agents mouillants, les agents mouillants, les stabilisants, les antimousses, les conservateurs, les viscosités, les liants, les adhésifs et les engrais.

8. La préparation selon la revendication 7, **caractérisée en ce qu'**elle contient de 0,01 à 99,09 % (p/p) du composé de formule générale (I) selon l'une quelconque des revendications 1 à 6, et de 99,09 à 0,01 % (p/p) d'au moins une substance auxiliaire, de préférence elle contient de 0,1 à 95 % (p/p) du composé de formule générale (I) et de 5 à 99,9 % de la au moins une substance auxiliaire.

9. La préparation selon la revendication 7 ou 8, **caractérisée en ce qu'**elle est dans la forme d'une solution, de préférence une solution aqueuse isotonique; suspension; dispersion; émulsion; poudre; poussière; granulé; crème; gel; suspension huileuse ou formulation enrobée.

10. Utilisation du composé de formule générale (I) selon l'une quelconque des revendications précédentes 1 à 6 et/ou de la préparation selon l'une quelconque des revendications précédentes 7 à 9 comme inhibiteur de la cytokinine oxydase dans la production de plantes cultivées.

11. Utilisation du composé de formule générale (I) selon l'une quelconque des revendications précédentes 1 à 6 et/ou de la préparation selon l'une quelconque des revendications précédentes 7 à 9 pour inhiber le stress dans les plantes entières, les organes végétaux, des tissus végétaux et/ou des cellules végétales.

12. Utilisation du composé de formule générale (I) selon l'une quelconque des revendications précédentes 1 à 6 et/ou de la préparation selon l'une quelconque des revendications précédentes 7 à 9 pour la stimulation de la germination des graines, de la croissance des plantules et la croissance et le développement des plantes.
